# EUROPEAN PATENT SPECIFICATION

(11) **EP 1 410 801 B1**
(45) Date of publication and mention of the grant of the patent: **10.04.2013**
(21) Application number: 03026256.2
(22) Date of filing: 26.01.1998
(51) Int. Cl.: A61K 38/08, C07K 7/14

(54) **Method for promoting hematopoietic cell proliferation and differentiation**
Verfahren zur Förderung der hämatopoietischen Zellproliferation und Differenzierung
Procédé favorisant la prolifération et la différentiation des cellules hématopoitiques

(30) Priority: 28.01.1997 US 36507 P; 08.05.1997 US 46859 P; 28.10.1997 US 63684 P; 31.10.1997 US 63910 P; 18.11.1997 US 65612 P; 26.11.1997 US 66593 P; 23.01.1998 US 12400
(43) Date of publication of application: 21.04.2004
(62) Divisional of application: 98908448.8
(73) Proprietor: UNIVERSITY OF SOUTHERN CALIFORNIA, Los Angeles, CA 90007-4344 (US)
(72) Inventor: Rodgers, Kathleen, E., Long Beach, CA 90815 (US); diZerega, Gere, San Luis Obispo, CA 93401 (US)
(74) Representative: Schnappauf, Georg

(56) References cited:
- WO-A-95/08337
- WO-A-96/39164
- US-A- 5 015 629
- MRUG M ET AL: "ANGIOTENSIN II FACILITIES ERYTHROPOIETIN MEDIATED PROLIFERATION OF EARLY ERYTHROID PROGENITORS" BLOOD, W.B. SAUNDERS, PHILADELPHIA, VA, US, vol. 88, November 1996 (1996-11), page 646A XP000944720 ISSN: 0006-4971
- BRANTLEY R R JR ET AL: "Blockade of angiotensin (A) II AT-1 receptor lowers hematocrits in post-transplant erythrocytosis (PTE)" JOURNAL OF THE AMERICAN SOCIETY OF NEPHROLOGY, vol. 7, no. 9, 1996, page 1930 XP002270162 29th Annual Meeting of the American Society of Nephrology;New Orleans, Louisiana, USA; November 3-6, 1996 ISSN: 1046-6673
- MRUG M ET AL: "Angiotensin II stimulates proliferation of normal early erythroid progenitors." JOURNAL OF CLINICAL INVESTIGATION, (1997 NOV 1) 100 (9) 2310-4, XP002077069

### SEQUENCE LISTING

<110> University of Southern California
<120> Method for Promoting Hematopoietic and Mesenchymal Cell Proliferation and Differentiation
<130> 150-71 T1
<140>
   <141>
<150>US19970036507P
   <151>1997-01-28
<150>US19970046859P
   <151>1997-05-08
<150>US19970063684P
   <151>1997-10-28
<150>US19970063910P
   <151>1997-10-31
<150>US19970065612P
   <151>1997-11-18
<150>US19970066593P
   <151>1997-11-26
<150>US19980012400
   <151>1998-01-23
<160> 38
<170> PatentIn Ver. 2.0
<210> 1
   <211> 8
   <212> PRT
   <213> Artificial Sequence
<220>
   <221> human
   <223> Description of Artificial Sequence:AII
<400> 1
   Asp Arg Val Tyr Ile His Pro Phe
   1               5
<210> 2
   <211> 7
   <212> PRT
   <213> Artificial Sequence
<220>
   <221> human
   <223> Description of Artificial Sequence:AII (2-8)
<400> 2
   Arg Val Tyr Ile His Pro Phe
   1               5
<210> 3
   <211> 6
   <212> PRT
   <213> Artificial Sequence
<220>
   <221> human
   <223> Description of Artificial Sequence:AII (3-8)
<400> 3
   Val Tyr Ile His Pro Phe
   1               5
<210> 4
   <211> 7
   <212> PRT
   <213> Artificial Sequence
<220>
   <221> human
   <223> Description of Artificial Sequence:AII (1-7)
<400> 4
   Asp Arg Val Tyr Ile His Pro
    1                5
<210> 5
   <211> 6
   <212> PRT
   <213> Artificial Sequence
<220>
   <221> human
   <223> Description of Artificial Sequence:AII (2-7)
<400> 5
   Arg Val Tyr Ile His Pro
    1                5
<210> 6
   <211> 5
   <212> PRT
   <213> Artificial Sequence
<220>
   <221> human
   <223> Description of Artificial Sequence:AII (3-7)
<400> 6
   Val Tyr Ile His Pro
    1               5
<210> 7
   <211> 4
   <212> PRT
   <213> Artificial Sequence
<220>
   <221> human
   <223> Description of Artificial Sequence:AII (5-8)
<400> 7
   Ile His Pro Phe
    1
<210> 8
   <211> 6
   <212> PRT
   <213> Artificial Sequence
<220>
   <221> human
   <223> Description of Artificial Sequence:AII (1-6)
<400> 8
   Asp Arg Val Tyr Ile His
   1 5
<210> 9
   <211> 5
   <212> PRT
   <213> Artificial Sequence
<220>
   <221> human
   <223> Description of Artificial Sequence:AII (1-5)
<400> 9
   Asp Arg Val Tyr Ile
   1 5
<210> 10
   <211> 4
   <212> PRT
   <213> Artificial Sequence
<220>
   <221> human
   <223> Description of Artificial Sequence:AII (1-4)
<400> 10
   Asp Arg Val Tyr
   1
<210> 11
   <211> 3
   <212> PRT
   <213> Artificial Sequence
<220>
   <221> human
   <223> Description of Artificial Sequence:AII (1-3)
<400> 11
   Asp Arg Val
   1
<210> 12
   <211> 7
   <212> PRT
   <213> Artificial Sequence
<220>
   <221> human
   <223> Description of Artificial Sequence:AII analogue
<220>
   <221> MOD_RES
   <222> (2)
   <223> Nle
<400> 12
   Arg Xaa Tyr Ile His Pro Phe
   1 5
<210> 13
   <211> 7
   <212> PRT
   <213> Artificial Sequence
<220>
   <221> human
   <223> Description of Artificial Sequence:AII analogue
<220>
   <221> MOD_RES
   <222> (4)
   <223> Nle
<400> 13
   Arg Val Tyr Xaa His Pro Phe
   1 5
<210> 14
   <211> 3
   <212> PRT
   <213> Artificial Sequence
<220>
   <221> human
   <223> Description of Artificial Sequence:AII (6-8)
<400> 14
   His Pro Phe
   1
<210> 15
   <211> 5
   <212> PRT
   <213> Artificial Sequence
<220>
   <221> human
   <223> Description of Artificial Sequence:AII (4-8)
<400> 15
   Tyr Ile His Pro Phe
   1 5
<210> 16
   <211> 7
   <212> PRT
   <213> Artificial Sequence
<220>
   <223> Description of Artificial Sequence:AII analogue class
<220>
   <221> UNSURE
   <222> (1)
   <223> Xaa at position 1 can be Arg, Lys, Ala, Orn, Ser, MeGly, D-Arg, or D-Lys
<220>
   <221> UNSURE
   <222> (2)
   <223> Xaa at position 2 can be Val, Ala, Leu, Nle, Ile, Gly, Pro, Aib, Acp, or Tyr
<220>
   <221> UNSURE
   <222> (4)
   <223> Xaa at position 4 can be Ile, Ala, Leu, Nle, Val, or Gly
<400> 16
   Xaa Xaa Tyr Xaa His Pro Phe
    1               5
<210> 17
   <211> 7
   <212> PRT
   <213> Artificial Sequence
<220>
   <221> human
   <223> Description of Artificial Sequence:AII analogue
<400> 17
   Arg Val Tyr Gly His Pro Phe
    1               5
<210> 18
   <211> 7
   <212> PRT
   <213> Artificial Sequence
<220>
   <221> human
   <223> Description of Artificial Sequence:AII analogue
<400> 18
   Arg Val Tyr Ala His Pro Phe
    1               5
<210> 19
   <211> 8
   <212> PRT
   <213> Artificial Sequence
<220>
   <221> human
   <223> Description of Artificial Sequence:AII analogue 1
<400> 19
   Asp Arg Val Tyr Val His Pro Phe
   1 5
<210> 20
   <211> 8
   <212> PRT
   <213> Artificial Sequence
<220>
   <221> human
   <223> Description of Artificial Sequence:AII analogue 2
<400> 20
   Asn Arg Val Tyr Val His Pro Phe
   1 5
<210> 21
   <211> 11
   <212> PRT
   <213> Artificial Sequence
<220>
   <221> human
   <223> Description of Artificial Sequence:AII analogue 3
<400> 21
   Ala Pro Gly Asp Arg Ile Tyr Val His Pro Phe
   1 5 10
<210> 22
   <211> 8
   <212> PRT
   <213> Artificial Sequence
<220>
   <221> human
   <223> Description of Artificial Sequence:AII analogue 4
<400> 22
   Glu Arg Val Tyr Ile His Pro Phe
   1 5
<210> 23
   <211> 8
   <212> PRT
   <213> Artificial Sequence
<220>
   <221> human
   <223> Description of Artificial Sequence:AII analogue 5
<400> 23
   Asp Lys Val Tyr Ile His Pro Phe
   1 5
<210> 24
   <211> 8
   <212> PRT
   <213> Artificial Sequence
<220>
   <221> human
   <223> Description of Artificial Sequence:AII analogue 6
<400> 24
   Asp Arg Ala Tyr Ile His Pro Phe
   1 5
<210> 25
   <211> 8
   <212> PRT
   <213> Artificial Sequence
<220>
   <221> human
   <223> Description of Artificial Sequence:AII analogue 7
<400> 25
   Asp Arg Val Thr Ile His Pro Phe
   1 5
<210> 26
   <211> 8
   <212> PRT
   <213> Artificial Sequence
<220>
   <221> human
   <223> Description of Artificial Sequence:AII analogue 8
<400> 26
   Asp Arg Val Tyr Leu His Pro Phe
   1 5
<210> 27
   <211> 8
   <212> PRT
   <213> Artificial Sequence
<220>
   <221> human
   <223> Description of Artificial Sequence:AII analogue 9
<400> 27
   Asp Arg Val Tyr Ile Arg Pro Phe
   1 5
<210> 28
   <211> 8
   <212> PRT
   <213> Artificial Sequence
<220>
   <221> human
   <223> Description of Artificial Sequence:AII analogue 10
<400> 28
   Asp Arg Val Tyr Ile His Ala Phe
   1 5
<210> 29
   <211> 8
   <212> PRT
   <213> Artificial Sequence
<220>
   <221> human
   <223> Description of Artificial Sequence:AII analogue 11
<400> 29
   Asp Arg Val Tyr Ile His Pro Tyr
   1 5
<210> 30
   <211> 8
   <212> PRT
   <213> Artificial Sequence
<220>
   <221> human
   <223> Description of Artificial Sequence:AII analogue 12
<400> 30
   Pro Arg Val Tyr Ile His Pro Phe
   1 5
<210> 31
   <211> 8
   <212> PRT
   <213> Artificial Sequence
<220>
   <221> human
   <223> Description of Artificial Sequence:AII analogue 13
<400> 31
   Asp Arg Pro Tyr Ile His Pro Phe
   1 5
<210> 32
   <211> 8
   <212> PRT
   <213> Artificial Sequence
<220>
   <221> human
   <223> Description of Artificial Sequence:AII analogue 14
<220>
   <221> MOD_RES
   <222> (4)
   <223> PHOSPHORYLATION
<400> 32
   Asp Arg Val Tyr Ile His Pro Phe
   1 5
<210> 33
   <211> 8
   <212> PRT
   <213> Artificial Sequence
<220>
   <221> human
   <223> Description of Artificial Sequence:AII analogue 15
<220>
   <221> MOD_RES
   <222> (3)
   <223> Nle
<400> 33
   Asp Arg Xaa Tyr Ile His Pro Phe
   1 5
<210> 34
   <211> 8
   <212> PRT
   <213> Artificial Sequence
<220>
   <221> human
   <223> Description of Artificial Sequence:AII analogue 16
<220>
   <221> MOD_RES
   <222> (5)
   <223> Nle
<400> 34
   Asp Arg Val Tyr Xaa His Pro Phe
   1 5
<210> 35
   <211> 9
   <212> PRT
   <213> Artificial Sequence
<220>
   <221> human
   <223> Description of Artificial Sequence:AII analogue 17
<220>
   <221> MOD_RES
   <222> (4)
   <223> homo Ser
<400> 35
   Asp Arg Val Xaa Tyr Ile His Pro Phe
   1 5
<210> 36
   <211> 8
   <212> PRT
   <213> Artificial Sequence
<220>
   <221> human
   <223> Description of Artificial Sequence:p-aminophenylalanine 6 AII
<220>
   <221> MOD_RES
   <222> (6)
   <223> p-aminophenylalanine
<400> 36
   Asp Arg Val Tyr Ile Xaa Pro Phe
   1 5
<210> 37
   <211> 10
   <212> PRT
   <213> Artificial Sequence
<220>
   <221> human
   <223> Description of Artificial Sequence:angiotensin I
<400> 37 210> 38
<211> 8
   <212> PRT
   <213> Artificial Sequence
<220>
   <221> human
   <223> Description of Artificial Sequence:Ile8-AII
<400> 38

## Description

### Cross Reference

This application is a Continuation of a U.S. Application filed on January 23, 1998, Application No. to be assigned; and a Continuation-In-Part of U.S. Provisional Application Nos. 60/036,507, filed January 28, 1997; 60/046,859, filed May 8, 1997; 60/063,684 filed October 28, 1997; 60/063,910 filed October 31, 1997; 60/065,612 filed November 18, 1997; and 60/066,593 filed November 26, 1997.

### Field of the Invention

This present invention relates to methods and kits for use in accelerating the proliferation and differentiation of hematopoietic and mesenchymal cells.

### Background of the Invention

Bone marrow contains pluripotent stem cells that are capable of reconstituting either the hematopoietic system or a wide range of mesenchymal tissues. The mechanisms by which hematopoietic and mesenchymal stem cells produce a range of lineage-specific cell types are quite dissimilar.

### a. The Hematopoietic System

The hematopoietic system is composed of a multitude of cell generations ranging from the terminally differentiated to very primitive hematopoietic lineage-specific cells, including a multipotent, self-renewing hematopoietic stem cell with long-term repopulating capability (HPC). (Traycoff, et al., Experimental Hematology 24:299-306, 1996). HPC are pluripotent lineage-specific cells that possess the ability to terminally differentiate into hematopoietic lineage-specific cells (HSC). Hematopoiesis is an ongoing process, and therefore HPC must provide a continuous source of HSC, which in turn can differentiate into red cells; platelets, monocytes, granulocytes and lymphocytes. (Prockop, Science 276:71-74, 1997). HPC proliferate either by "self-renewal", to produce HPC-type progeny cells, or with accompanying differentiation, to produce HLSC. (Traycoff, et al., *supra*).

HPC transplantation therapy has been successful for a variety of malignant and inherited diseases and also provides myelopoietic support for patients undergoing high-dose chemotherapy or radiotherapy. (Emerson, Blood 87:3082-3088, 1996). However, stem cell transplantation has been limited by several features. First, acquiring a sufficient quantity of stem cells to achieve benefit after transfusion requires either extensive, operative bone marrow harvests or extensive pheresis procedures. (Emerson, *supra*). Next, even under these circumstances, only a limited number of useful cells is obtained. Finally, mature blood cell regeneration after transfusion is slow, so that little direct therapeutic benefit is seen for periods of 1 to 3 weeks. (Emerson, *supra*).

The development of *in vitro* culture techniques for hematopoietic cells combined with technologies for isolating relatively pure populations of HPC and HLSC has made possible their *ex vivo* expansion. (Alcorn and Holyoake, Blood Reviews 10:167-176, 1996, which is incorporated by reference herein). Successful *ex vivo* expansion of HPC, both by self-renewal and proliferation with differentiation, promises many clinical benefits, such as reduction of the number and duration of leucapheresis procedures required for autologous transplantation, thus reducing the risk of disease contamination in the apheresis products. (Alcorn and Holyoake, supra). Furthermore, *ex vivo* expansion may render inadequate HPC populations in peripheral blood and umbilical cord blood sufficient for autologous transplantation and adult allogeneic transplantation respectively. Finally, *ex vivo* expansion of HPC will greatly increase their utility as gene therapy vehicles. (Alcorn and Holyoake, *supra*). Similarly, *ex vivo* expansion of HLSC promises substantial clinical benefits, such as re-infusion of expanded populations of myeloid precursor cells to reduce the period of obligate neutropenia following autologous transplantation, the generation of natural killer cells for use in adoptive immunotherapy protocols, generation of megakaryocyte precursors to alleviate post-transplant-associated thrombocytopenia and more efficient generation of delivery systems for gene therapy. (Alcorn and Holyoake, *supra*).

Human bone marrow, umbilical cord blood, and peripheral blood lineage-specific cells mobilized by chemotherapy and/or cytokine treatment have been shown to be effective sources of HPC for transplantation following the administration of high-dose therapy to treat malignancy. (Holyoake, et al., Blood 87:4589-4595, 1996). Whatever the source of hematopoietic cells, most studies have used cultured cell populations selected on the basis of HPC-specific surface antigens, such as CD34. These cells can be readily obtained by a number of techniques. (Alcorn and Holyoake, *supra*). The results of several clinical trials using *ex vivo* expanded hematopoietic cells suggests that a fairly small number of HPC cultured *ex vivo* under appropriate conditions can initiate hematologic reconstitution. (Emerson, *supra*).

Survival and proliferation of HPC in *ex vivo* culture requires a combination of synergizing growth factors; the choice of cytokine/growth factor combination and culture system used will largely determine the fate of cells used to initiate the culture. (Alcorn and Holyoake, supra). *In vivo,* blood cell production is thought to be regulated locally by interactions of hematopoietic stem cells with a variety of cell-bound and secreted factors produced by adjacent bone marrow stromal cells. (Alcorn and Holyoake, *supra*). The addition of growth factors and cytokines to the culture medium is intended to compensate for the absence of stroma-associated activities. Growth factors and cytokines that have been shown to increase production of HPC (in various combinations) include granulocyte colony-stimulating factor (G-CSF), granulocyte/macrophage colony stimulating factor (GM-CSF), stem cell factor (SCF), macrophage colony-stimulating factor (M-CSF), and interleukins 1, 3, 6, and 11 (Reviewed in Takaku, J. Cancer Res. Clin. Oncol. 121:701-709, 1995; Holyoake, et al., *supra*)*.* Conversely, inclusion of macrophage inhibitory protein-1α (MIP-1α), tumor necrosis factor α (TNF-α) or transforming growth factor β (TGFβ) in most expansion cultures reported to date results in decreased HPC and HLSC yields. (Emerson, *supra*).

A great deal of effort has gone into defining the optimal conditions for *ex vivo* culture of hematopoietic cells. Improved methods that increase of *ex vivo* proliferation rate of HPC will greatly increase the clinical benefits of HPC transplantation. This is true both for increased "self-renewal", which will provide a larger supply of HPC capable of reconstituting the entire hematopoietic system, and for proliferation with differentiation, which will provide a larger supply of lineage-specific cells. Similarly, methods that increase *in vivo* proliferation of HPC will enhance the utility of HPC transplantation therapy by rapidly increasing local concentrations of HPC (and HLSC) in the bone marrow. Furthermore, methods that result in the differentiation of HPC and HLSC are useful in providing populations of specific cell types for use in cell therapy.

Transfection of mammalian HPC has been accomplished. (Larochelle, et al., Nature Medicine 2:1329-1337, 1996). Thus, methods that increase the proliferation of HPC and HLSC are also useful in rapidly providing a large population of transfected cells for use in gene therapy.

### b. Mesenchymal stem cells

Mesenchymal stem cells (MSC) are pluripotent progenitor cells that possess the ability to differentiate into a variety of mesenchymal tissue, including bone, cartilage, tendon, muscle, marrow stroma, fat and dermis as demonstrated in a number of organisms, including humans (Bruder, et al., J. Cellul. Biochem. 56:283-294 (1994). The formation of mesenchymal tissues is known as the mesengenic process, which continues throughout life, but proceeds much more slowly in the adult than in the embryo (Caplan, Clinics in Plastic Surgery 21:429-435 (1994). The mesengenic process in the adult is a repair process but involves the same cellular events that occur during embryonic development (Reviewed in Caplan, 1994, *supra*). During repair processes, chemoattraction brings MSC to the site of repair where they proliferate into a mass of cells that spans the break. These cells then undergo commitment and enter into a specific lineage pathway (differentiation), where they remain capable of proliferating. Eventually, the cells in the different pathways terminally differentiate (and are no longer able to proliferate) and combine to form the appropriate skeletal tissue, in a process controlled by the local concentration of tissue-specific cytokines and growth factors (Caplan, 1994, *supra*).

Recently, it has been hypothesized that the limiting factor for MSC-based repair processes is the lack of adequate numbers of responsive MSC at the repair site (Caplan, 1994, *supra*). Thus, it has been suggested that by supplying a sufficient number of MSC to a specific tissue site the repair process can be controlled, since the repair site will supply the appropriate exposure to lineage-specific growth factors and differentiation molecules (Caplan, 1994, *supra*). Towards this end, several animal studies have demonstrated the feasibility of using autologous MSC for repair of various defects associated with mesenchymal tissue. (For review, see Caplan, et al., in The Anterior Cruciate Ligament: Current and Future Concepts, ed. D.W. Jackson, Raven Press, Ltd. NY pp.405-417 (1993). Recent work has demonstrated the feasibility of collection, ex *vivo* expansion in culture, and intravenous infusion of MSC in humans (Lazarus, et al., Bone Marrow Transplantation 16:557-564 (1995); Caplan and Haynesworth, U.S. Patent No. 5,486,359, hereby incorporated by reference in its entirety). Further, MSC of animal origin have been transfected with retroviruses and have achieved high level gene expression both *in vitro* and *in vivo* (Allay, et al., Blood 82:477A (1993). Thus, the manipulation of MSC via such techniques seems a promising tool for reconstructive therapies and may be useful for gene therapy.

MSC therapy can serve as a means to deliver high densities of repair-competent cells to a defect site when adequate numbers of MSC and MSC lineage-specific cells are not present *in vivo*, especially in older and/or diseased patients. In order to efficiently deliver high densities of MSC to a defect site, methods for rapidly producing large numbers of MSC are necessary. MSC have been exposed to a number of growth factors *in vitro,* but only platelet-derived growth factor (PDGF) showed mitotic activity (Caplan et al., 1994, *supra*). Methods that increase the *ex vivo* Proliferation rate of MSC will greatly increase the utility of MSC therapy. Similarly, methods that increase *in vivo* proliferation rate of MSC will enhance the utility of MSC therapy by rapidly increasing local concentrations of MSC at the repair site.

Furthermore, methods that enhance the proliferation rate of lineage-specific descendants of MSC, including but not limited to bone marrow stromal cells, osteoclasts, chondrocytes, and adipocytes, will enhance the therapeutic utility of MSC therapy by increasing the concentration of lineage-specific cell types at appropriate repair sites.

Both angiotensin II (AII) and angiotensin III (AIII) have been shown to accelerate wound healing (US 5,015,629 A and WO 95/08337 A, respectively). WO 9639164 A teaches that angiotensin II receptor subtype 2 (AT2) agonists can be used to accelerate wound healing. Besides, Mrug et al. propose that AII enhances CD34+ erythroid differentiation and that this effect is angiotensin II receptor subtype (AT1)-mediated (Mrug et al., Blood 88:646A, 1996). Brantley et al. administered an AT1 antagonist to renal transplant patients and observed reduction of hematocrit in some of the patients (Brantley et al. Journal of the American Society of Nephrology (7):1930, 1997). Mrug et al. found that AII increased numbers of CD34+ erythroid progenitors and that an AT1 antagonist abolished this effect (Mrug et al., J. Clin. Invest. (100):2310-4, 1997).

### Summary of the Invention

In one aspect, the present invention provides methods that promote hematopoietic stem and lineage-specific cell proliferation and differentiation, by contacting the cells with a peptide whose sequence is selected from the group consisting of Val-Tyr-Ile-His-Pro-Phe (SEQ ID NO:3), Asp-Arg-Val-Tyr-Ile-His-Pro (SEQ ID NO:4), Arg-Val-Tyr-Ile-His-Pro (SEQ ID NO:5), Val-Tyr-Ile-His-Pro (SEQ ID NO:6), Ile-His-Pro-Phe (SEQ ID NO:7), Asp-Arg-Val-Tyr-Ile-His (SEQ ID NO:8), Asp-Arg-Val-Tyr-Ile (SEQ ID NO:9), Asp-Arg-Val-Tyr (SEQ ID NO:10), Asp-Arg-Val (SEQ ID NO:11), Asp-Arg-Pro-Tyr-Ile-His-Pro-Phe (SEQ ID NO:31) and Asp-Arg-Val-Tyr-Ile-His-Pro-Ile (SEQ ID NO:38) either alone or in combination with other growth factors and cytokines.

### Brief Description of the Drawings

**Figure 1** is a graph showing the effect of AII on the phagocytic capability of murine macrophages.
**Figure 2** is a graph showing the effect of AII on the phagocytic capability of rat macrophages.
**Figure 3** is a graph showing the effect of AII on respiratory burst function in rat peritoneal macrophages.
**Figure 4** is a graph showing the effect of AII on respiratory burst function in human PBMC.
**Figure 5** is a graph showing the effect of AII(1-7) on respiratory burst function in rat peritoneal macrophages.
**Figure 6** is a graph showing the effect of GSD 24B on respiratory burst function in rat peritoneal macrophages.
**Figure 7** is a graph showing the effect of GSD 22A on respiratory burst function in rat peritoneal macrophages.
**Figure 8** is a graph showing the effect of GSD 28 on respiratory burst function in rat peritoneal macrophages.
**Figure 9** is a graph showing the effect of AII on proliferation in response to pokeweed mitogen.
**Figure 10** is a graph showing the effect of AII on rat bone marrow cultures.
**Figure 11** is a graph showing the effect of AII on rat bone marrow cultures.
**Figure 12** is a graph showing the effect of AII on murine HSC cultures.
**Figure 13** is a graph showing the effect of AII on murine HSC cultures.
**Figure 14** is a graph showing the effect of AII on murine HSC cultures.
**Figure 15** is a graph showing the effect of AII on murine HSC cultures.
**Figure 16** is a graph showing the effect of AII on murine HSC cultures.
**Figure 17** is a graph showing the effect of All on murine HSC cultures.
**Figure 18** is a graph showing the effect of AII(I-7) on MSC proliferation.
**Figure 19** is a graph showing the effect of GSD 22A on MSC proliferation.
**Figure 20** is a graph showing the effect of GSD 24B on MSC proliferation.
**Figure 21** is a graph showing the effect of GSD 28 on MSC proliferation.
**Figure 22** is a graph showing the effect of AII on alkaline phosphatase expression by MSC.
**Figure 23** is a graph showing the effect of AII without colony stimulating factors on murine HPC cultures.
**Figure 24** is a graph showing the effect of AII without colony stimulating factors on murine HPC cultures.
**Figure 25** is a graph showing the effect of AII without colony stimulating factors on murine HPC cultures.
**Figure 26** is a graph showing the effect of AII and AII analogues without colony stimulating factors on murine HPC cultures.
**Figure 27** is a graph showing the effect of AII and AII analogues without colony stimulating factors on murine HPC cultures.
**Figure 28** is a graph showing the effect of AII and AII analogues without colony stimulating factors on murine HPC cultures.
**Figure 29** is a graph showing the effect of AII and AII analogues without colony stimulating factors on murine HPC cultures.
**Figure 30** is a graph showing the effect of AII and AII analogues without colony stimulating factors on murine HPC cultures.
**Figure 31** is a graph showing the effect of AII and AII analogues without colony stimulating factors on murine HPC cultures.
**Figure 32** is a graph showing the effect of AII and AII analogues without colony stimulating factors on murine HPC cultures.
**Figure 33** is a graph showing the effect of AII and AII analogues without colony stimulating factors on murine HPC cultures.

### Detailed description of the invention

As defined herein, the term "HPC" refers to any hematopoietic pluripotent progenitor cells capable of giving -rise to' a wide variety of differentiated hematopoietic cell types. Cell types within this definition include, but are not limited to CD34* bone marrow mononuclear cells (BMMC) (Berardi, et al., Blood 86:2123-2129, 1995), PBSC (Fritsch, et al., Bone Marrow Transplantation 17:169-178, 1996), cobblestone area forming cells (CAFC) (Lemieux, et al., Blood 86:1339-1347, 1995) and 5-FU BM cells (Alcorn and Holyoake, Blood Reviews 10:167-176, 1996). As defined herein, the term "HLSC" refers to hematopoietic lineage-specific cells, and includes the progeny of HPC that are committed to a cell-specific differentiation pathway, as well as fully differentiated hematopoietic cells. As defined herein, mesenchymal stem cells (MSC) are pluripotent progenitor cells that possess the ability to differentiate into a variety of mesenchymal tissue, including bone, cartilage, tendon, muscle, marrow stroma, fat and dermis, and include, but are not limited to, cells such as those described in Caplan and Haynesworth, U.S. Patent No. 5,486,359. As defined herein, "proliferation" encompasses both cell self renewal and cellular proliferation with accompanying differentiation. As defined herein "differentiation" includes both entry into a specific lineage pathway and functional activation of differentiated cells.

U.S. Patent No. 5,015,629 to DiZerega describes a method for increasing the rate of healing of wound tissue, comprising the application to such tissue of angiotensin II (AII) in an amount which is sufficient for said increase. The application of AII to wound tissue significantly increases the rate of wound healing, leading to a more rapid re-epithelialization and tissue repair. The term AII refers to an octapeptide present in humans and other species having the sequence Asp-Arg-Val-Tyr-De-His-Pro-Phe [SEQ ID NO:1]. The biological formation of angiotensin is initiated by the action of renin on the plasma substrate angiotensinogen (Clouston et al., Genomics 2:240-248 (1988); Kageyama et al, Biochemistry 23:3603-3609; Ohkubo et al., Proc. Natl. Acad. Sci. 80:2196-2200 (1983); The substance so formed is a decapeptide called angiotensin I (AI) which is converted to AII by the converting enzyme angiotensinase which removes the C-terminal His-Leu residues from AI [SEQ ID NO: 38]. AII is a known pressor agent and is commercially available.

Studies have shown that AII increases mitogenesis and chemotaxis in cultured cells, that are involved in wound repair, and also increases their release of growth factors and extracellular matrices (diZerega, U.S. Patent No. 5,015,629; Dzau et. al., J. Mol. Cell. Cardiol. 21:S7 (Supp III) 1989; Berk et. al., Hypertension 13:305-14 (1989); Kawahara, et al., BBRC 150:52-9 (1988); Naftilan, et al., J. Clin. Invest. 83:1419-23 (1989); Taubman et al., J. Biol. Chem. 264:526-530 (1989); Nakahara, et al., BBRC 184:811-8 (1992); Stouffer and Owens, Circ. Res. 70:820 (1992); Wolf, et al., Am. J. Pathol. 140:95-107 (1992); Bell and Madri, Am. J. Pathol. 137:7-12 (1990). In addition, AII was shown to be angiogenic in rabbit corneal eye and chick chorioallantoic membrane models (Fernandez, et al., J. Lab. Clin. Med. 105:141 (1985); LeNoble, et al., Eur. J. Pharmacol. 195:305-6 (1991). Therefore, AII may accelerate wound repair through increased neovascularization, growth factor release, reepithelialization and/or production of extracellular matrix. However, it is not known in the literature as to whether angiotensinogen, AI, AI analogues, AI fragments and analogues thereof, An, AII analogues and fragments thereof, or AII receptor agonists would be useful in accelerating the proliferation and differentiation of hematopoietic and lineage-specific cells.

A peptide agonist selective for the AT2 receptor (AII has 100 times higher affinity for AT2 than AT1) has been identified. This peptide is p-aminophenylanine-AII ["(p-NH₂-Phe)6-AII"], Asp-Arg-Val-Tyr-Ile-Xaa-Pro-Phe [SEQ ID NO.36] wherein Xaa is p-NH₂-Phe (Speth and Kim, BBRC 169:997-1006 (1990). This peptide gave binding characteristics comparable to AT2 antagonists in the experimental models tested (Catalioto, et al., Eur. J. Pharmacol. 256:93-97 (1994); Bryson, et al., Eur. J. Pharmacol. 225:119-127 (1992).

The effects of AII receptor and AII. receptor antagonists have been examined in two experimental models of vascular injury and repair which suggest that both AII receptor subtypes (AT1 and AT2) play a role in wound healing (Janiak et al., Hypertension 20:737-45 (1992); Prescott, et al., Am. J. Pathol. 139:1291-1296 (1991); Kauffinan, et al., Life Sci. 49:223-228 (1991); Viswanathan, et al., Peptides 13:783-786 (1992); Kimura, et al., BBRC 187:1083-1090 (1992).

Many studies have focused upon AII(1-7) (AII residues 1-7 or other fragments of AII to evaluate their activity. AII(1-7) elicits some, but not the full range of effects elicited by AII. Pfeilschifter, et al., Eur. J. Pharmacol. 225:57-62 (1992); Jaiswal, et al., Hypertension 19(Supp. II):II-49-II-55 (1992); Edwards and Stack, J. Pharmacol. Exper. Ther. 266:506-510 (1993); Jaiswal, et al., J. Pharmacol. Exper. Ther. 265:664-673 (1991); Jaiswal, et al., Hypertension 17:1115-1120 (1991); Portsi, et a., Br. J. Pharmacol. 111:652-654 (1994).

The active AII analogues, fragments of AII and analogues thereof of particular interest in accordance with the present invention comprise a peptide whose sequence is selected from the group consisting of Val-Tyr-Ile-His-Pro-Phe (SEQ ID NO:3), Asp-Arg-Val-Tyr-Ile-His-Pro (SEQ ID NO:4), Arg-Val-Tyr-Ile-His-Pro (SEQ ID NO:5), Val-Tyr-Ile-His-Pro (SEQ ID NO:6), Ile-His-Pro-Phe (SEQ ID NO:7), Asp-Arg-Val-Tyr-Ile-His (SEQ ID NO:8), Asp-Arg-Val-Tyr-Ile (SEQ ID NO:9), Asp-Arg-Val-Tyr (SEQ ID NO:10), Asp-Arg-Val (SEQ ID NO:11), Asp-Arg-Pro-Tyr-Ile-His-Pro-Phe (SEQ ID NO:31) and Asp-Arg-Val-Tyr-Ile-His-Pro-Ile (SEQ ID NO:38).

In the above formulas, the standard three-letter abbreviations for amino acid residues are employed. In the absence of an indication to the contrary, the L-form of the amino acid is intended.

The polypeptides may be synthesized by any conventional method, including, but not limited to, those set forth in J. M. Stewart and J. D. Young, Solid Phase Peptide Synthesis, 2nd ed., Pierce Chemical Co., Rockford, III. (1984) and J. Meienhofer, Hormonal Proteins and Peptides, Vol. 2, Academic Press, New York, (1973) for solid phase synthesis and E. Schroder and K. Lubke, The Peptides, Vol. 1, Academic Press, New York, (1965) for solution synthesis. The disclosures of the foregoing treatises are incorporated by reference herein.

In general, these methods involve the sequential addition of protected amino acids to a growing peptide chain (U.S. Patent No. 5,693,616). Normally, either the amino or carboxyl group of the first amino acid and any reactive side chain group are protected. This protected amino acid is then either attached to an inert solid support, or utilized in solution, and the next amino acid in the sequence, also suitably protected, is added under conditions amenable to formation of the amide linkage. After all the desired amino acids have been linked in the proper sequence, protecting groups and any solid support are removed to afford the crude polypeptide. The polypeptide is desalted and purified, preferably chromatographically, to yield the final product.

Preferably, peptides are synthesized according to standard solid-phase methodologies, such as may be performed on an Applied Biosystems Model 430A peptide synthesizer (Applied Biosystems, Foster City, Calif.), according to manufacturer's instructions. Other methods of synthesizing, peptides or peptidomimetics, either by solid phase methodologies or in liquid phase, are well known to those skilled in the art.

Although AII has been shown to increase the proliferation of a number of cell types *in vitro,* it does not necessarily increase the proliferation of all cell types. Studies have shown that AII accelerates cellular proliferation through the production of transforming growth factor β (TGFβ) (Gibbons et al., J. Clin. Invest. 90:456-461 (1992). Thus, since only PDGF is known to be mitogenic for MSC, an ability of AII to effect MSC proliferation would be unexpected. Furthermore, as Emerson (*supra*) has shown that inclusion of TGF-β in most expansion cultures resulted in a decreased HPC and HLSC yield, it is unexpected that AII, through the action of TGF-β, would be of benefit in such situations. No studies have reported that AII has an effect on the differentiation of either HPC or MSC.

In one aspect of the present invention, a method of increasing *in vitro* and *ex vivo* HPC and HLSC proliferation by exposure to a peptide whose sequence is selected from the group consisting of the peptides represented by SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:31 and SEQ ID NO:38. is disclosed. Experimental conditions for the isolation, purification, *ex vivo* growth and *in vivo* mobilization of HPC and HLSC have been reported (Berardi, et al., Blood 86(6):2123-2129, 1995; Fritsch, et al., Bone Marrow Transplantation 17:169-178, 1996; LaRochelle, et al., Nature Medicine 12:1329-1337, 1996; Traycoff, et al., Experimental Hematology 24:299-306, 1996; Holyoake, et al., Blood 87:4589-4595, 1996; Lemieux, et al., Blood 86:1339-1347, 1995 ; Talmadge, et al., Bone Marrow Transplantation 17:101-109, 1996; Bodine, et aL, Blood 88:89-97, 1996; all references hereby incorporated by reference herein.)

In one embodiment of the invention, HPC are isolated from bone marrow, peripheral blood or umbilical cord blood. HPC is then selected for in these samples. HPC-enriched samples are cultured under appropriate growth conditions, in the presence of a peptide whose sequence is selected from the group consisting of the peptides represented by SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:31 and SEQ ID NO:38. HPC proliferation is assessed at various time points during culture.

In a preferred embodiment, HPC and HLSC are isolated from bone marrow aspirates from the posterior iliac crest (Caplan and Haynesworth, U.S. Patent No. 5,486,359). CD34⁺ HPC are isolated from the aspirate by attaching a biotinylated monoclonal antibody specific for CD34 (available from Becton Dickinson, Sunnyvale, CA, USA) to a streptavidin affinity column (Ceprate SC; CellPro, Bothell, WA, USA) and passing the aspirate through the column, followed by appropriate column washing and stripping, according to standard techniques in the art. CD34⁺ HPC are suspended in culture medium and incubated in the presence of, preferably, between about 0.1 ng/ml and about 1 mg/ml angiotensinogen, AI, AI analogues, AI fragments and analogues thereof, AII, AII analogues, AII fragments and analogues thereof and/or AII AT₂ type 2 receptor agonists. The cells are expanded for a period of between 8 and 21 days and cellular proliferation with accompanying differentiation is assessed via phase microscopy following standard methylcellulose colony formation assays (Berardi, et al., *supra*) at various points during this time period. Similarly, "self-renewal" of HPC is assessed periodically by reactivity to an antibody directed against CD34⁺.

In a further referred embodiment, HPC that have been cultured in the presence of a peptide whose sequence is selected from the group consisting of the peptides represented by SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:31 and SEQ ID NO:38 are used for autologous transplantation, to reconstitute a depleted hematopoietic system. Prior to transplantation, the cells are rinsed to remove all traces of culture fluid, resuspended in an appropriate medium and then pelleted and rinsed several times. After the final rinse, the cells are resuspended at between 0.7 x 10⁶ and 50 x 10⁶ cells per ml in an appropriate medium and reinfused into a subject through intravenous infusions. Following transplantation, subject peripheral blood samples are evaluated for increases in the number of HPC, HLSC, and more mature blood cells at various time points by standard flow cytometry and cell sorting techniques. (Talmadge, et al., *supra*)*.*

In another aspect of the present invention a peptide whose sequence is selected from the group consisting of the peptides represented by SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:31 and SEQ ID NO:38 used to increase *in vivo* HPC, HLSC, MSC and mesenchymal lineage-specific cell proliferation. For use in increasing proliferation of HPC, and HLSC a peptide whose sequence is selected from the group consisting of the peptides represented by SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:31 and SEQ ID NO:38 may be administered by any suitable route, including orally, parentally, by inhalation spray, rectally, or topically in dosage unit formulations containing conventional pharmaceutically acceptable carriers, adjuvants, and vehicles. The term parenteral as used herein includes, subcutaneous, intravenous, intramuscular, intrasternal, intratendinous, intraspinal, intracranial, intrathoracic, infusion techniques or intraperitoneally.

In a further embodiment of the present invention, a method of increasing *in vivo* HPC, and HLSC, proliferation by exposure to a peptide whose sequence is selected from the group consisting of the peptides represented by SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:31 and SEQ ID NO:38 is disclosed, either in the presence or absence of other growth factors and cytokines. Examples of such growth factors and cytokines include, but are not limited to lymphokines, interleukins - 1, 2, 3, 4, 5, 6, 7, 8, 9, 10, 11, granulocyte colony-stimulating factor, granulocyte/macrophage colony stimulating factor, macrophage colony-stimulating factor, tumor necrosis factor, epidermal growth factor, fibroblast growth factor, platelet derived growth factor, transforming growth factor beta, and stem cell factor. A peptide whose sequence is selected from the group consisting of the peptides represented by SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:31 and SEQ ID NO:38 may be made up in a solid form (including granules, powders or suppositories) or in a liquid form (*e.g.*, solutions, suspensions, or emulsions). The compounds of the invention may be applied in a variety of solutions. Suitable solutions for use in accordance with the invention are sterile, dissolve sufficient amounts of the peptides, and are not harmful for the proposed application. In this regard, the compounds of the present invention are very stable but are hydrolyzed by strong acids and bases. The compounds of the present invention are soluble in organic solvents and in aqueous solutions at pH 5-8.

A peptide whose sequence is selected from the group consisting of the peptides represented by SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:31 and SEQ ID NO:38 may be subjected to conventional pharmaceutical operations such as sterilization and/or may contain conventional adjuvants, such as preservatives, stabilizers, wetting agents, emulsifiers, buffers etc.

While a peptide whose sequence is selected from the group consisting of the peptides represented by SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:31 and SEQ ID NO:38 can be administered as the sole active pharmaceutical agent, they can also be used in combination with one or more other agents. When administered as a combination, the therapeutic agents can be formulated as separate compositions that are given at the same time or different times, or the therapeutic agents can be given as a single composition.

For administration, a peptide whose sequence is selected from the group consisting of the peptides represented by SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:31 and SEQ ID NO:38 is ordinarily combined with one or more adjuvants appropriate for the indicated route of administration. The compounds may be admixed with lactose, sucrose, starch powder, cellulose esters of alkanoic acids, stearic acid, talc, magnesium stearate, magnesium oxide, sodium and calcium salts of phosphoric and sulphuric acids, acacia, gelatin, sodium alginate, polyvinylpyrrolidine, and/or polyvinyl alcohol, and tableted or encapsulated for conventional administration. Alternatively, the compounds of this invention may be dissolved in saline, water, polyethylene glycol, propylene glycol, carboxymethyl cellulose colloidal solutions, ethanol, corn oil, peanut oil, cottonseed oil, sesame oil, tragacanth gum, and/or various buffers. Other adjuvants and modes of administration are well known in the pharmaceutical art. The carrier or diluent may include time delay material, such as glyceryl monostearate or glyceryl distearate alone or with a wax, or other materials well known in the art.

Formulations suitable for topical administration include liquid or semiliquid preparations suitable for penetration through the skin (*e.g*., liniments, lotions, ointments, creams, or pastes) and drops suitable for administration to the eye, ear, or nose.

The dosage regimen for increasing *in vivo* proliferation or differentiation of HPC, and HLSC a peptide whose sequence is selected from the group consisting of the peptides represented by SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:31 and SEQ ID NO:38 is based on a variety of factors, including the type of injury, the age, weight, sex, medical condition of the individual, the severity of the condition, the route of administration, and the particular compound employed. Thus, the dosage regimen may vary widely, but can be determined routinely by a physician using standard methods. Dosage levels of the order of between 0.1 ng/kg and 1 mg/kg of a peptide whose sequence is selected from the group consisting of the peptides represented by SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:31 and SEQ ID NO:38 per body weight are useful for all methods of use disclosed herein.

The treatment regime will vary depending on the disease being treated, based on a variety of factors, including the type of injury, the age, weight, sex, medical condition of the individual, the severity of the condition, the route of administration, and the particular compound employed. For example, a peptide whose sequence is selected from the group consisting of the peptides represented by SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:31 and SEQ ID NO:38 is administered to an oncology patient for up to 30 days prior to a course of radiation therapy. The therapy is administered for 1 to 6 times per day at dosages as described above. In another example, in order to mobilize hematopoietic lineage-specific cells for donation by plasmapheresis the therapy is for up to 30 days for 1 to 6 times per day.

In a preferred embodiment of the present invention, a peptide whose sequence is selected from the group consisting of the peptides represented by SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:31 and SEQ ID NO:38 is preferably between about 0.1 ng/ml and about 1 mg/ml administered twice daily. For topical administration, the active ingredient may comprise from 0.0001% to 10% w/w, *e.g*., from 1% to 2% by weight of the formulation, although it may comprise as much as 10% w/w, but preferably not more than 5% w/w, and more preferably from 0.1% to 1% of the formulation.

The present invention, by providing a method for enhanced proliferation of HPC and HLSC, will greatly increase the clinical benefits of HPC transplantation. This is true both for increased "self-renewal", which will provide a larger supply of HPC capable of reconstituting the entire hematopoietic system, and for proliferation with differentiation, which will provide a larger supply of lineage-specific cells, for more rapid reconstitution of mature, functioning blood cells. Similarly, methods that increase *in vivo* proliferation of HPC will enhance the utility of HPC transplantation therapy by rapidly increasing local concentrations of HPC (and HLSC) in the bone marrow, and thereby more rapidly producing functioning blood cells.

The method of the present invention also increases the potential utility by more efficiently providing a large number of such cells for transfection, and also, by providing a more efficient means to rapidly expand transfected HPC, and HLSC.

The present invention may be better understood with reference to the accompanying examples that are intended for purposes of illustration only and should not be construed to limit the scope of the invention, as defined by the claims appended hereto.

### Comparative Example 1. Macrophage Differentiation: Phagocytosis

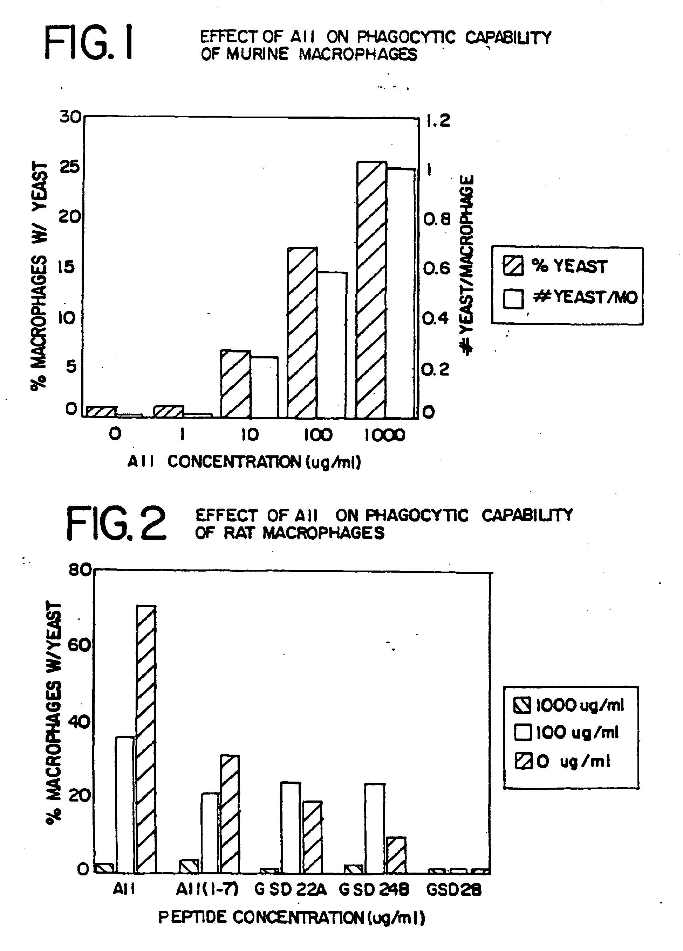

Resident peritoneal macrophages have very little phagocytic activity. Exposure of macrophages to inflammatory or activating agents will increase this macrophage function. Resident peritoneal macrophages were harvested from C57B1/6 mice or Sprague Dawley rats and resuspended at a concentration of 1 x 10⁶ cells/ml in phosphate buffered saline (PBS). Five separate 0.5 ml cell aliquots were placed on a glass coverslip in a 35 mm petri dish. Prior to incubation, either 0.5 ml of PBS, AII, or AII analogues or fragments at between 1-1000 ug/ml final concentration was added to the individual cover slips. The dishes containing the cover slips were then incubated at 37°C for 4 hours, after which the cover slips were washed 3 to 6 times with PBS. Opsonized yeast particles (Sigma Chemical Co.) (yeast opsonized with adult serum from the same species as that under study) were added to the cover slips and incubated for 2 hours, after which the cover slips were again washed with PBS and inverted onto a glass slide. The number of macrophages that ingested yeast and the number of yeast per macrophage ingested was then determined microscopically. At least 100 macrophages per coverslip were counted. The data from this study are summarized in Tables 3 and 4 and Figures 1 and 2. Table 5 describes the AII analogues and fragments used in these studies.

Exposure to 10 µg/ml or greater AII tremendously increased the phagocytic capability of peritoneal macrophages. Less than 1% of cells were phagocytic in the resident population (0.01 yeast per cell observed). After exposure to AII this increased to over 25% phagocytic at the highest concentration with on average 1 yeast observed per macrophage (25 fold increase in the number of macrophages able to phagocytose and a 100-fold increase in the number of particles phagocytized).

As shown in Table 4, both concentration of the peptides tested (with the exception of GSD 28) elevated that phagocytic capability of rat macrophages. However, none of the analogues resulted in the magnitude of an effect observed with AII. This suggests that AII and, to a lesser extent, AII analogues will stimulate macrophage differentiation to an elicited or activated state, which leads to the ingestion and clearance of bacteria and cellular debris.

**Table 3. Effect of AII on the Phagocytic Capability of Murine Peritoneal Macrophages**

| Concentration of AII (µg/ml) | | | | | |
|---|---|---|---|---|---|
| | 0 | 1 | 10 | 100 | 1000 |

| # Macrophages with Yeast | | | | | |
|---|---|---|---|---|---|
| Yeast # | | | | | |
| 0 | 100 | 100 | 100 | 100 | 100 |
| 1-3 | 1 | 1 | 3 | 10 | 20 |
| 4-6 | 0 | 0 | 4 | 10 | 20 |
| 7-9 | 0 | 0 | 0 | 0 | 0 |

| % Ingesting | | | | | |
|---|---|---|---|---|---|
| | 1 | 1 | 6.5 | 16.7 | 25.6 |

| # Yeast/MO | | | | | |
|---|---|---|---|---|---|
| | 0.01 | 0.01 | 0.24 | - 0.58 | 1.00 |

**Table 4. Effect of AII on the Phagocytic Capability of Rat Peritoneal Macrophages**

| | Number of Macrophages with Yeast | | | | | |
|---|---|---|---|---|---|---|
| | | (Yeast Number) | | % w/Yeast #/Cell | | |
| | 0 | 1-3 | 4-6 | ≥7 | | |
| (Peptide) µg/ml | | | | | | |
| | | | | | | |
| AII-0 | 88 | 2 | 0 | 0 | 2.2 | 0.04 |
| 100 | 90 | 30 | 20 | 0 | 35.7 | 1.14 |
| 1000 | 50 | 54 | 40 | 25 | 70.4 | 2.86 |

| AII(1-7) | | | | | | |
|---|---|---|---|---|---|---|
| 0 | 90 | 3 | 0 | 0 | 3.2 | 0.06 |
| 100 | 100 | 21 | 5 | 0 | 20.6 | 0.53 |
| 1000 | 90 | 30 | 10 | 0 | 30.8 | 0.85 |
| | | | | | | |

| GSD22A | | | | | | |
|---|---|---|---|---|---|---|
| 0 | 90 | 1 | 0 | 0 | 1.1 | 0.02 |
| 100 | 100 | 26 | 5 | 0 | 23.7 | 0.59 |
| 1000 | 100 | 22 | 1 | 0 | 18.7 | 0.40 |

| GSD 24-B | | | | | | |
|---|---|---|---|---|---|---|
| 0 | 100 | 2 | 0 | 0 | 2.0 | 0.04 |
| 100 | 100 | 25 | 5 | 0 | 23.1 | 0.58 |
| 1000 | 100 | 10 | 0 | 0 | 9.1 | 0.18 |
| | | | | | | |

| GSD28 | | | | | | |
|---|---|---|---|---|---|---|
| 0 | 100 | 1 | 0 | 0 | 1.0 | 0.02 |
| 100 | 100 | 1 | 0 | 0 | 1.0 | 0.02 |
| 1000 | 100 | 1 | 0 | 0 | 1.0 | 0.02 |

**Table 5. Designation for Analogues**

| Name | Abbreviation | Sequence |
|---|---|---|
| GSD 28 | Ile⁸-AII | DRVYIHPI |
| GSD 24B | Pro³-AII | DRPYIHPF |
| GSD 22A | Ala⁴-AII | RVYAHPF |
| AII(1-7) | | DRVYIHP |

### Example 2 Leukocyte Differentiation: Respiratory Burst

The respiratory burst of leukocytes (macrophages and polymorphonuclear neutrophils) is one component of the mediator system used to kill bacteria. As with phagocytosis, the level of this respiratory burst activity in resident macrophages is low. With differentiation, either to an elicited (inflammatory) or activated state, this functional activity is significantly elevated. Studies were conducted to assess the effect of *in vitro* exposure of murine or rat peritoneal macrophages and human peripheral blood mononuclear cells (PBMC) to various concentrations of AII on the capacity to generate hydrogen peroxide via the respiratory burst system. For the human studies, five different donors were examined.

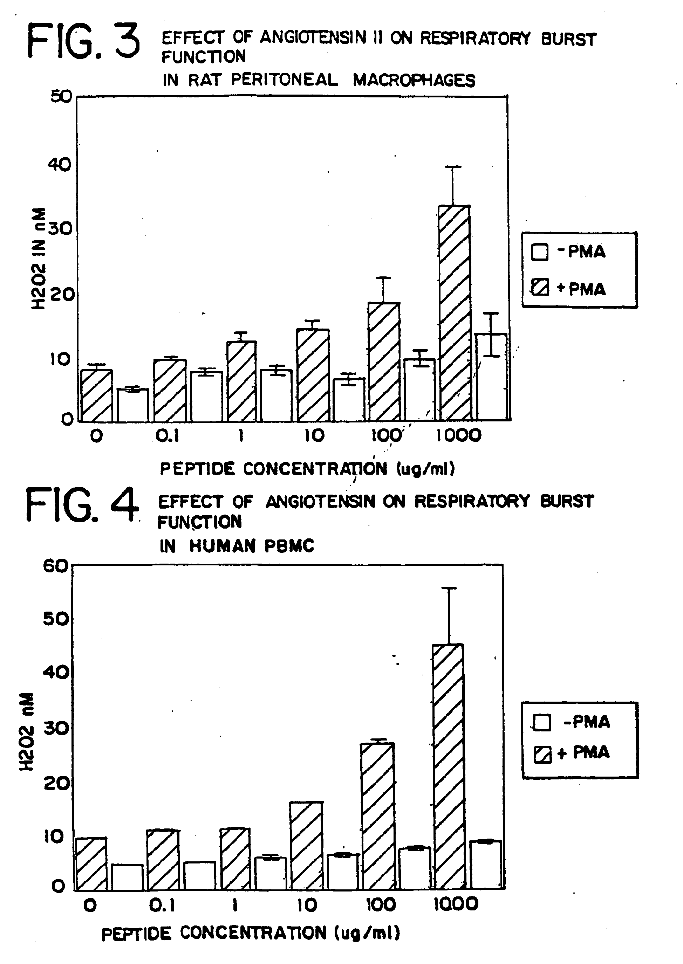

The murine or rat peritoneal cells were harvested by lavage with 5-15 ml of cold PBS with 0.5% bovine serum albumin. The human PBMC were harvested by venipuncture from normal human volunteers and isolated from peripheral blood by Ficoll Hypaque density centrifugation. After isolation, the cells were resuspended at 1 x 10⁶ cells/ml and placed at 100µL per well into 96 well plates. The cells were incubated with various concentrations of AII or AII analogues for 4 hours at 37°C. The cells were then preloaded with a fluorescent probe for hydrogen peroxide, 2,7 dichlorofluorescein acetate, which is nonfluorescent in the absence of hydrogen peroxide. Fifteen minutes later, 10 ng/ml of phorbol myristate acetate (+PMA) or PBS (-PMA) was added to stimulate the production of hydrogen peroxide. One hour after stimulation the level of fluorescence produced was measured on a Cytofluor 2350 multiwell fluorometer. Representative results from this study are shown in Figures 3-4.

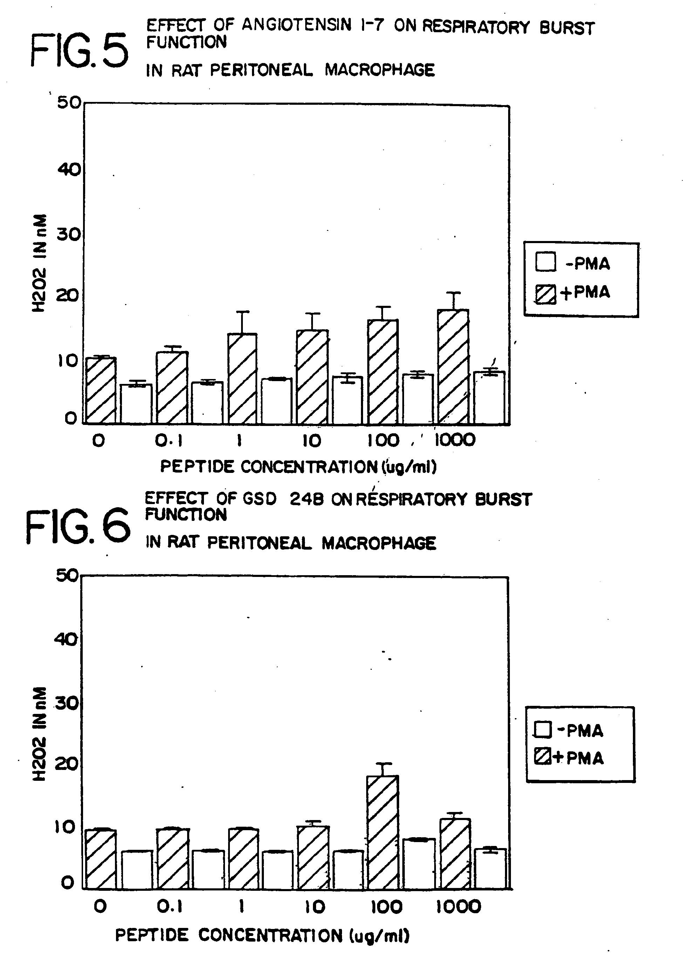

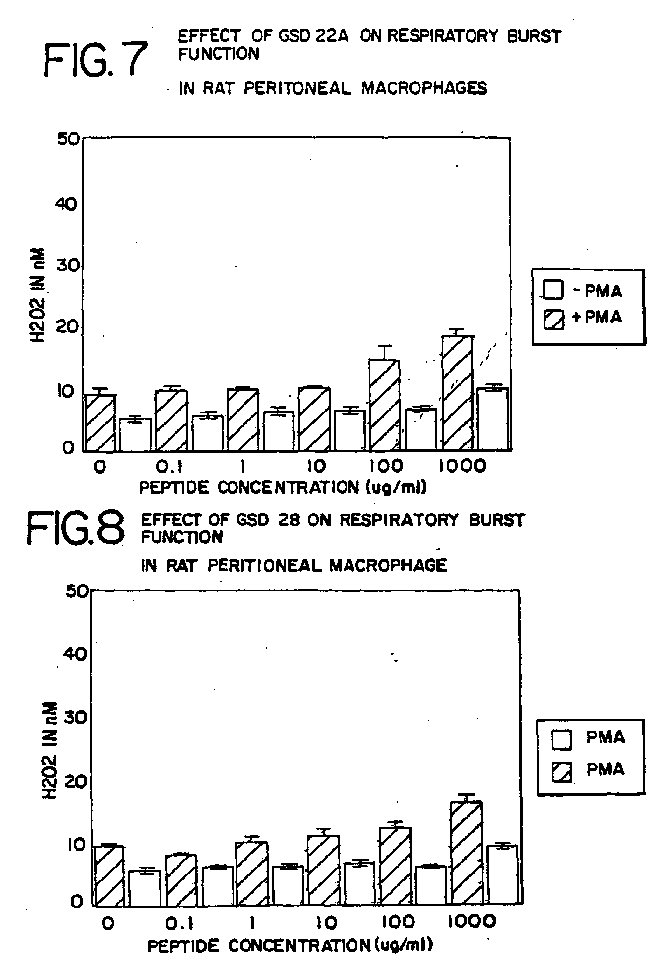

In the absence of PMA or peptide, no hydrogen peroxide production is observed. Some variability in the response to AII was seen (i.e. the concentration of AII necessary to increase the level of this function); however, in all studies AII increased the ability of leukocytes to generate hydrogen peroxide both alone and in response to stimulation with PMA. Further, the effect of pre-exposure to analogues of AII (GSD 22A, GSD 24B, GSD 28 and AII(1-7)) on the respiratory burst activity of PBMC was assessed (Figures 5-8). For all analogues, a much higher concentration of the peptide was needed to increase the respiratory burst activity. Up to 100 times more of these analogues were necessary; however, an increase in the respiratory burst capacity was observed for all analogues tested. The analogues were able to stimulate that function both in the presence and absence of PMA. These data indicate that AII was able to stimulate the differentiation of monocytes/macrophages from three species.

### Example 3. Proliferative response of human lymphocytes

Upon stimulation of lymphocytes with mitogen or antigen, these cells undergo blastogenesis and proliferation. In the absence of such stimuli, proliferation is seldom observed. One method to measure cellular proliferation in a short term assay is via measurement of the amount of the nucleotide thymidine that is incorporated into newly synthesized DNA. The effect of AII on the proliferation of human PBMC in the presence and absence of pokeweed mitogen ("PWM") was assessed.

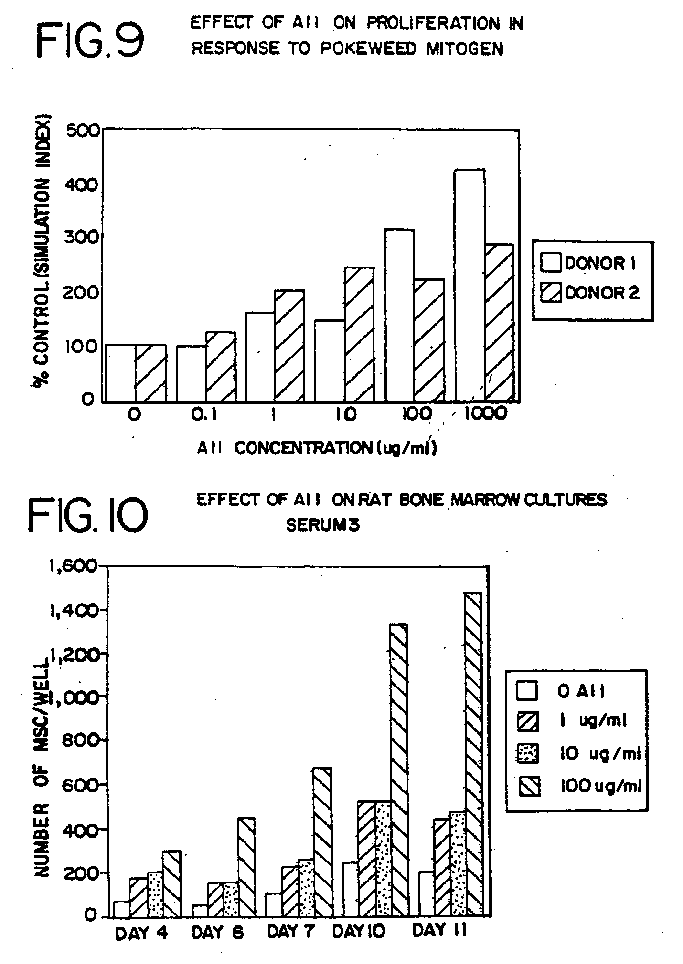

Human PBMC were collected from normal volunteers and isolated via Ficoll Hypaque (Sigma Chemical, St. Louis) density centrifugation. After isolation of the buffy coat, the cells were washed 3X to remove the Ficoll Hypaque, counted in trypan blue (0.01%) and resuspended at a concentration of 1 x 10⁶ cells/ml in RPMI 1640 containing 10% fetal calf serum and antibiotics. A 100 µl aliquot of cells was added to each well. Thereafter, various concentrations (0.1 to 1000 µg/ml final concentration) of AII in RPMI 1640 containing 10% FCS and antibiotics were added to various wells in triplicate. To the appropriate wells, PWM (20 µg/ml final concentration) was added. These plates were incubated at 37°C in 5% CO₂ for 48 hours. Subsequently, 0.5 µCi of ³H-thymidine was added to each well, which were incubated at 37°C for an additional 24 hours prior to harvest by a multiwell automated sample harvester (Skatron) onto glass fiber filters. These filters were dried, placed in scintillation fluid and the amount of thymidine incorporated was determined by beta counting. The results are shown in Figure 9 and Table 6.

In the absence of mitogen, no increase in thymidine incorporation was observed after exposure to AII. However, in two separate experiments (cells from 2 different donors) AII was shown to increase the amount of thymidine incorporated in response to PWM. These data show that AII is able to increase the proliferation of cells from the hematopoietic lineage (e.g. lymphocytes).

**Table 6. Effect of AII on the Proliferation of Human PBMC**

| AII Concentration | Proliferation Index cpm ³H-Thymidine Incorporated (SI^{a}) | |
|---|---|---|
| | Experiment 1^{b} | Experiment 2 |
| 0 | 1146.3 (3.63) | 2022.0 (31.11) |
| 0.1 | 1329.0 (3.54) | 2135.0 (38.13) |
| 1 | 1411.2 (5.88) | 2996.0 (62.42) |
| 10 | 1427.0 (5.34) | 3946.0 (75.88) |
| 100 | 1552.1 (11.41) | 3747.0 (69.39) |
| 1000 | 1551.5 (15.48) | 4302.0 (89.63) |

| | | |
|---|---|---|
| "SI= Stimulation Index is calculated from the ration of the counts incorporated ^{a}SI= Stimulation Index is calculated from the ration of the counts incorporated into stimulated cultures divided by counts in cultures without mitogen (FCS only-basal proliferation). This is shown in the parentheses. ^{b}The effect of AII on the proliferation of PBMC from two separate donors are shown in this table. | | |

### Comparative Example 4 Rat Mesenchymal Stem Cell Proliferation

These studies were conducted to determine the effect that AII would have on the proliferation of MSC. Bone marrow cells were harvested from the femur and tibia of female Sprague Dawley rats by flushing the bones with Dulbecco's Minimal Essential Medium-High Glucose ("DMEM-HG") with a syringe and an 18 gauge needle. These cells were cultured in 24 well plates at 5 x 10³ cells/mm² in DMEM-HG containing selected lots of fetal calf serum (FCS) and antibiotics (complete medium) at 37°C incubator containing 5% CO₂ in air. Twenty-four hours after the initiation of the cultures the medium and nonadherent cells were aspirated and fresh medium was added. To each of these several wells, complete medium with (1 to 100 µg/ml) or without AII was added. The migration of cells from the clones and their proliferation was followed by microscopic examination. Every 4 days the old medium was removed and fresh medium was added to the cultures.

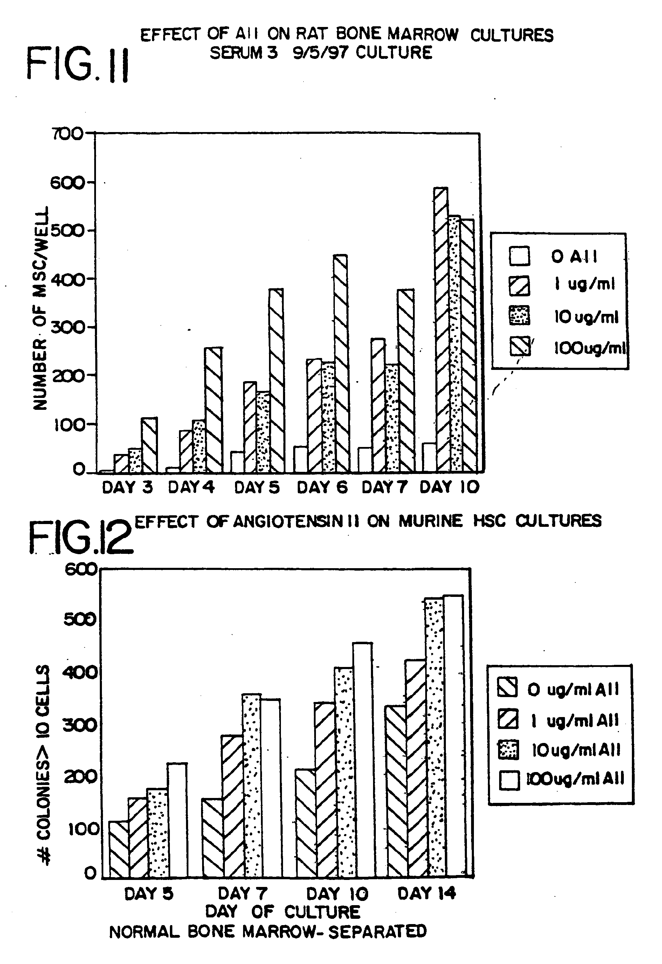

The data from these experiments are shown in Figures 10 and 11. Addition of AII to the cultures significantly increased the number of sites from which the MSC were migrating (CPU) and the size (number of cells) of the colonies formed. This occurred only in the presence of serum that in itself would support MSC growth, albeit to a lesser extent. As can be seen, AII caused an increase in the number of MSC in a concentration dependent manner at all time points examined in the presence of two different serum lots. These data support the hypothesis that AII can increase the proliferation of rat MSC.

### Example 5 Effect of Angiotensin II on Murine Lineage-specific Cells

HPC were harvested from C57B1/6 mice by immunomagnetic affinity chromatography and placed in semi-solid cultures with optimal growth medium. At various times after initiation of culture, the formation of colonies and the size of the colonies (number of cells/colony) was assessed microscopically.

Female C57B1/6 mice were purchased from Simonson and used as a source of bone marrow cells in this study. The bone marrow was harvested, either from untreated mice or from mice injected with 5-fluorouracil (5-FU) (3 mg/mouse; approximately 150 mg/kg) in the tail vein 48 hours before harvest, from the femur and tibia of mice by flushing with phosphate buffered saline (PBS), pH 7.4, containing 2% fetal bovine serum (FBS) with a 21-gauge needle. The eluant from the flushing was centrifuged and the pellet was resuspended at 4 x 10⁷ nucleated cells/ml in PBS containing PBS containing 2% FBS and 5% normal rat serum.

The reagents for immunomagnetic labeling were purchased from Stem Cell Technologies, Inc. (Vancouver, BC). Biotin-labeled monoclonal antibodies to the following murine lineage-specific cell surface antigens were included in a cocktail for HPC enrichment and used according to the manufacturer's instructions: CD5 (Ly-1), CD45-R (B220), CD11b (Mac-1), Myeloid Differentiation Antigen (Gr-1) and Erythroid Cells (TER 119) Ten µl of antibody cocktail was added for each of the 2 sets of bone marrow (normal and 5-FU-treated), mixed and allowed to incubate at 4°C for 15 minutes. The cells were then resuspended at 4 x 10⁷ cells/ml in PBS containing 2% FBS. The antibody cocktail was then washed out and 100 µl anti-biotin tetramer was added for each ml of cells. The suspension was mixed and incubated at 4°C for 15 minutes. Sixty µl of magnetic colloid was then added for each ml of cells, the combination was mixed and incubated at 4°C for 15 minutes to yield the immunomagnetically-labeled bone marrow cells.

A column containing a stainless steel matrix was prepared by washing the matrix with PBS followed by washing with PBS containing 2% protein. The immunomagnetically-labeled bone marrow cells were loaded onto the column and unlabeled cell-containing medium (enriched HPC) was collected in the flow through fraction at a flow rate of 0.2 ml/minute. Medium was added to the top of the column so that it did not run dry until 8 to 10 ml of enriched HPC were harvested. Approximately 2% of the cells loaded onto the column were isolated in the enriched HPC fractions.

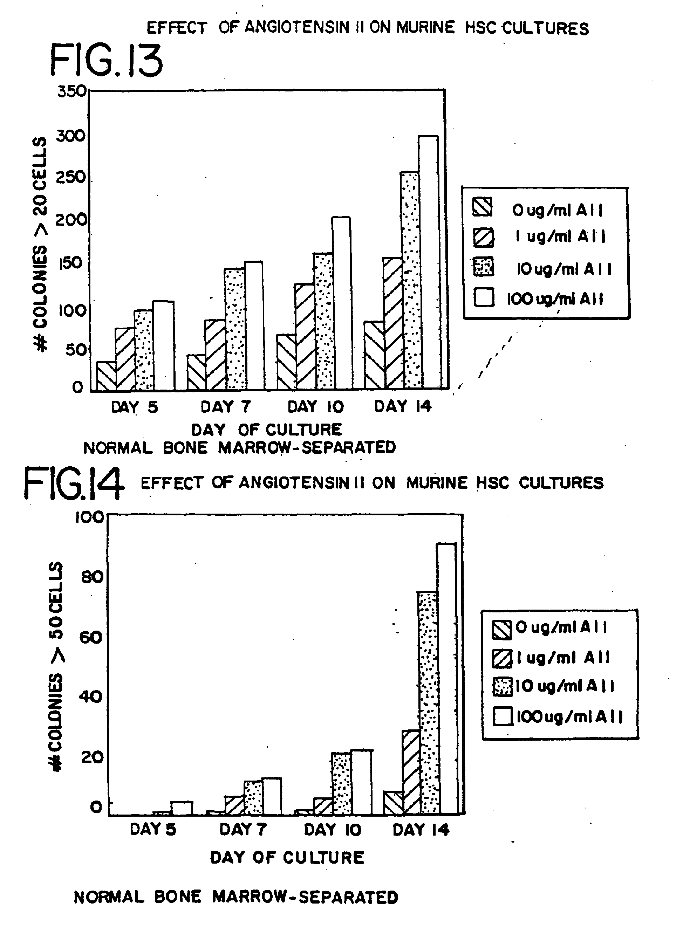

The enriched HPC cell fractions were diluted into a semi-solid medium containing 0.9% methylcellulose in alpha minimal essential medium (alpha MEM), 30% fetal calf serum, 1% bovine serum albumin, 10⁻⁴ M 2-mercaptoethanol, 2 mM L-glutamine, and 2% conditioned medium containing colony stimulating factors. The conditioned medium was supernatant from splenocyte cultures (1 x 10⁶ cells/ml) incubated for 48 hours in RPMI 1640 containing 10 µg/ml pokeweed mitogen (PWM), 10% FCS, and antibiotics. Various concentrations of AII, between 0 and 100 µg/ml were added in a small volume to the wells of microliter dishes, to which between 2 x 10⁴ cells/ml for the normal and 2.5 x 10° cells/ml for the 5-FU treated cells. The cells were incubated at 37°C and 5% CO₂ for 14 days. At day 14 only, macroscopic cell colonies were observed in the wells containing enriched HPC from untreated (normal) mice treated with 10 µg/ml (18 macroscopic colonies) and 100 µg/ml AII (10 macroscopic colonies). Microscopic evaluation of the cells was performed at various days after initiation of incubation, and the results are summarized in Figures 12-17.

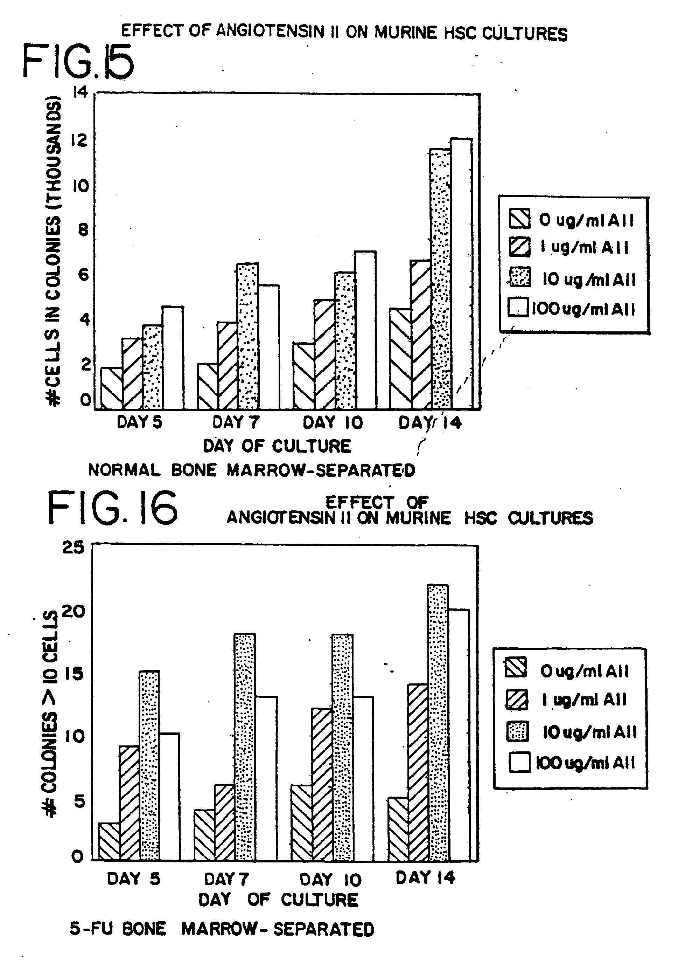

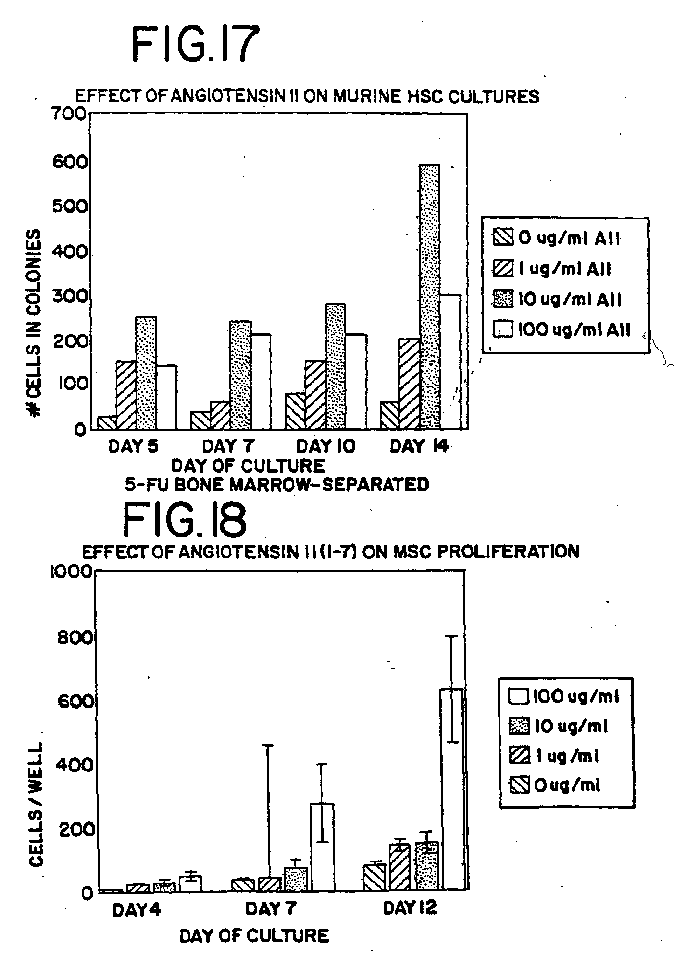

Figures 12-14 and 16 represent the number of colonies containing more than a certain number of cells/colony as a function of the duration and concentration of AII exposure (Figures 12-14 for normal cells; Figure 16 for 5-FU treated cells.) Figures 15 and 17 represents the number of cells per colony seen after incubation of bone marrow from normal or 5-FU treated mice with various concentrations of AII as a function of time. The results clearly demonstrate that HPC colony size increases proportionately with exposure to increased concentrations of AII, and thus that AII increases HPC proliferation.

### Comparative Example 6. Effect of AII Analogues and Fragments on Rat Mesenchymal Stem Cell Proliferation

These studies were conducted to determine the effect that inclusion of AII analogues and fragments in the cell culture of MSC would have on the proliferation of these cells. Bone marrow cells were harvested from the femur and tibia of female Sprague Dawley rats by flushing the bones with Dulbecco's Minimal Essential Medium-High Glucose ("DMEM-HG") with a syringe and an 18 gauge needle. These cells were cultured in 24 well plates at 5 x 10³ cells/mm² in DMEM-HG containing selected lots of fetal calf serum (FCS) and antibiotics (complete medium) at 37°C incubator containing 5% CO₂ in air. Twenty-four hours after the initiation of the cultures the medium and nonadherent cells were aspirated and fresh medium was added. To each of these several wells, complete medium with (1 to 100 µg/ml) or without AII analogues and fragments (see Table 5) was added. The migration of cells from the clones and their proliferation was followed by microscopic examination. Every 4 days the old medium was removed and fresh medium was added to the cultures.

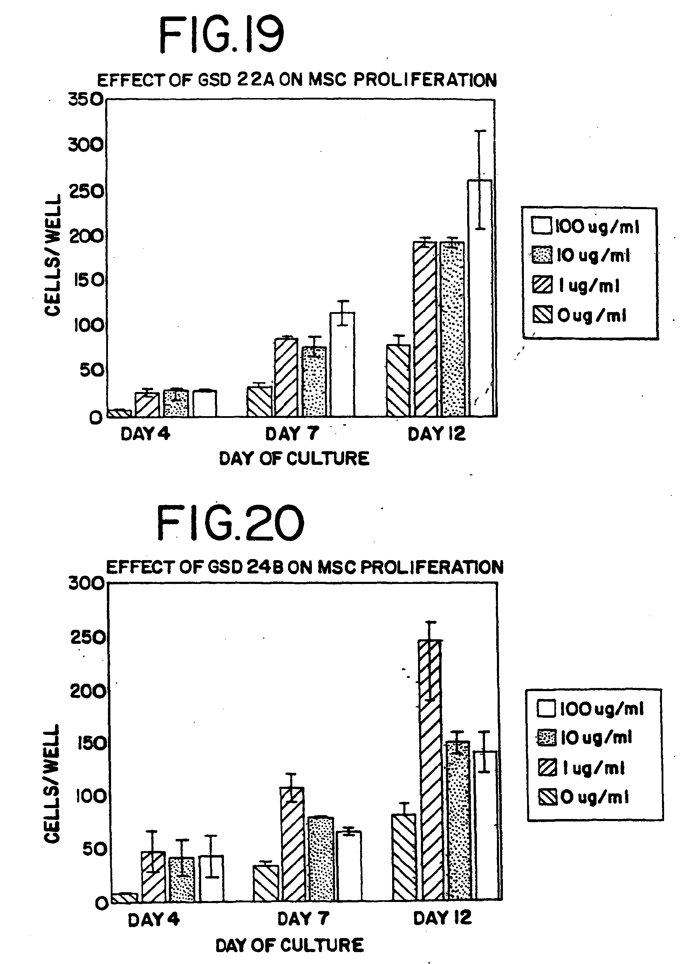

Addition of AII analogues or AlI fragments to these cultures had a profound effect on the number of sites from which MSC were migrating (CFU) and the size (number of cells) of the colonies formed. The results from these studies can be seen in Figures 18-21. As can be seen, AII analogues and fragments caused an increase in the number of MSC at all time points examined. These data indicate that AII analogues and fragments can increase the proliferation of rat MSC.

### Comparative 7. Proliferation of MSC Lineage-Specific Cells in the Presence of AII

Mesenchymal stem cells isolated from bone marrow and grown under appropriate conditions can express characteristics of multiple cell types, including cells involved in the generation of bone, cartilage, muscle and tendons. Osteogenic cells (cells that can form bone tissue) express the enzyme alkaline phosphatase when cultured in medium that drives them toward their osteogenic differentiation.

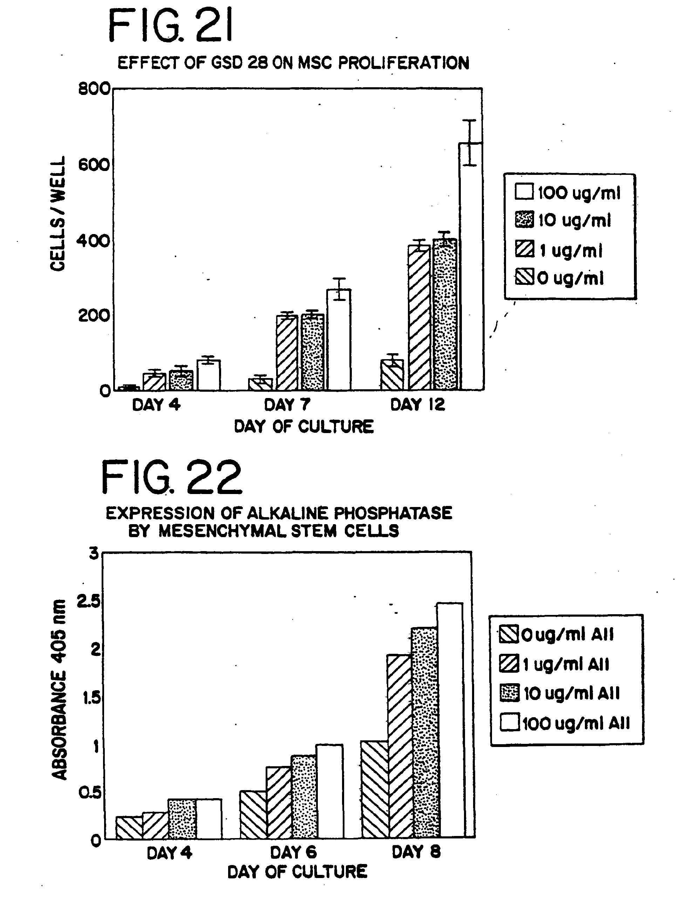

Bone marrow from female Sprague Dawley rats were harvested by flushing the femur with medium The cells were placed in culture dishes 9 cm² in diameter, allowed to adhere overnight, and then placed in DMEM-LG medium containing antibiotics and 10% fetal calf serum together with varying concentrations of AII. At various times after culture initiation, the cells were washed with Tyrode's buffer and placed in osteogenic medium (DMEM-LG containing 10% fetal calf serum, 100 nM dexamethasone and 0.05 mM ascorbic acid) for 4 days prior to assessment of the level of alkaline phosphatase activity per well. Briefly, the wells were washed with Tyrode's buffer and 1 ml alkaline phosphatase substrate solution (50 mM glycine, pH 10.5, containing 1 mM magnesium chloride and 2.5 mM p-nitrophenyl phosphate) to each well. Fifteen minutes after addition of this aqueous substrate, the buffer was removed from the culture and mixed with 1 ml of IN sodium hydroxide to stop the reaction. The absorbance of the resultant mixture at 405 nm was then determined via spectroscopy. The level of alkaline phosphatase activity is expressed as the level of absorbance per culture dish. These data are shown in Figure 22 and demonstrate that AII can accelerate the proliferation of cells that express alkaline phosphatase when placed in medium appropriate to induce osteogenic differentiation.

### Example 8 Effect of Angiotensin II on Murine Lineage-specific Cells

HPC were harvested from C57B1/6 mice by immunomagnetic affinity chromatography and placed in semi-solid cultures with optimal growth medium in the absence of colony stimulating factors. At various times after initiation of culture, the formation of colonies and the size of the colonies (number of cells/colony) was assessed microscopically.

Female C57B1/6 mice were purchased from Simonson and used as a source of bone marrow cells in this study. The bone marrow was harvested from the femur and tibia of mice by flushing with phosphate buffered saline (PBS), pH 7.4, containing 2% fetal bovine serum (FBS) with a 21-gauge needle. The eluant from the flushing was centrifuged and the pellet was resuspended at 4 x 10⁷ nucleated cells/ml in PBS containing PBS containing 2% FBS and 5% normal rat serum.

The reagents for immunomagnetic labeling were purchased from Stem Cell Technologies, Inc. (Vancouver, BC). Biotin-labeled monoclonal antibodies to the following murine lineage-specific cell surface antigens were included in a cocktail for HPC enrichment and used according to the manufacturer's instructions: CDS (Ly-1), CD45-R (B220), CD11b (Mac-1), Myeloid Differentiation Antigen (Gr-1) and Erythroid Cells (TER 119) Ten µl of antibody cocktail was added to the bone marrow, mixed and allowed to incubate at 4°C for 15 minutes. The cells were then resuspended at 4 x 10⁷ cells/ml in PBS containing 2% FBS. The antibody cocktail was then washed out and 100 µl anti-biotin tetramer was added for each ml of cells. The suspension was mixed and incubated at 4°C for 15 minutes. Sixty µl of magnetic colloid was then added for each ml of cells, the combination was mixed and incubated at 4°C for 15 minutes to yield the immunomagnetically-labeled bone marrow cells.

A column containing a stainless steel matrix was prepared by washing the matrix with PBS followed by washing with PBS containing 2% protein. The immunomagnetically-labeled bone marrow cells were loaded onto the column and unlabeled cell-containing medium (enriched HPC) was collected in the flow through fraction at a flow rate of 0.2 ml/minute. Medium was added to the top of the column so that it did not run dry until 8 to 10 ml of enriched HPC were harvested. Approximately 2% of the cells loaded onto the column were isolated in the enriched HPC fractions.

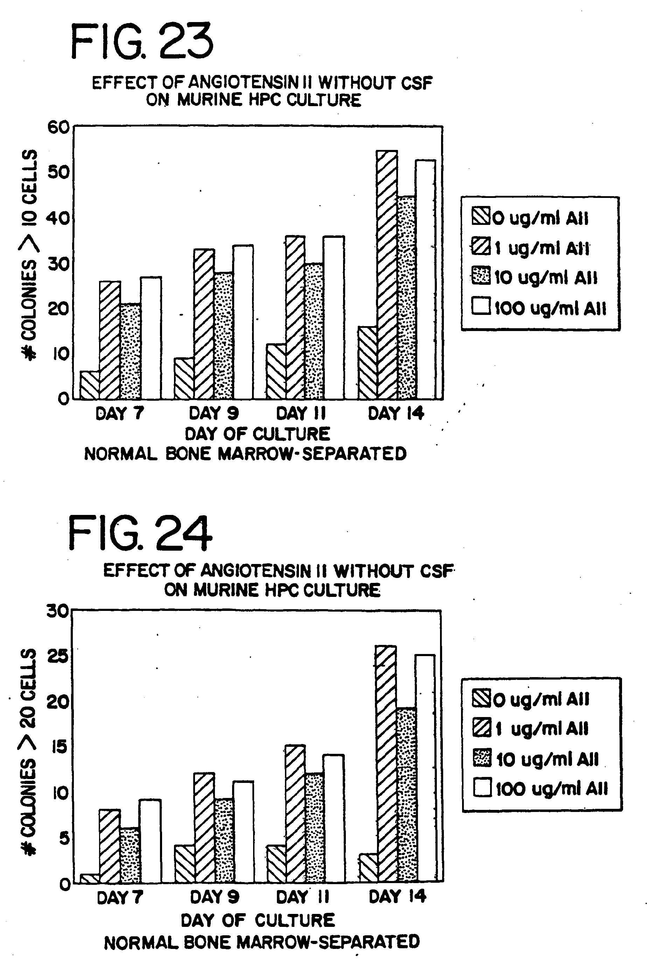

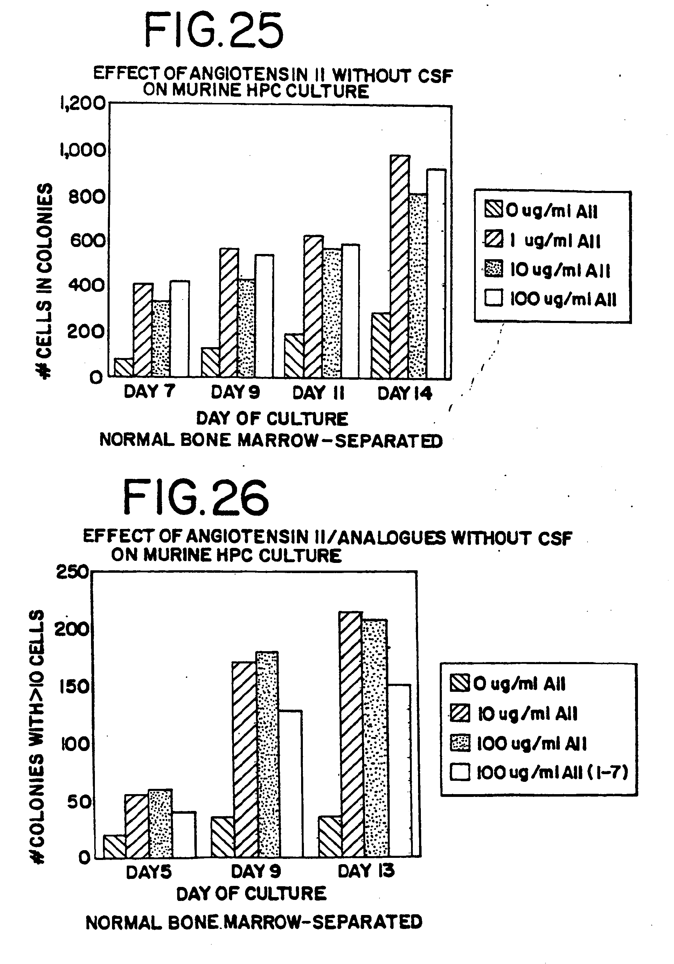

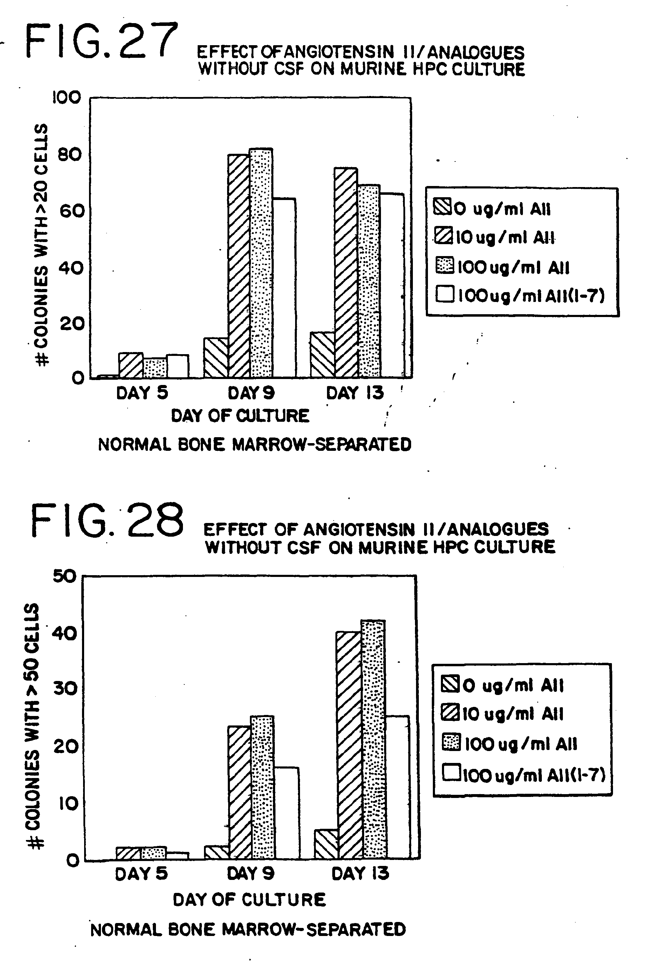

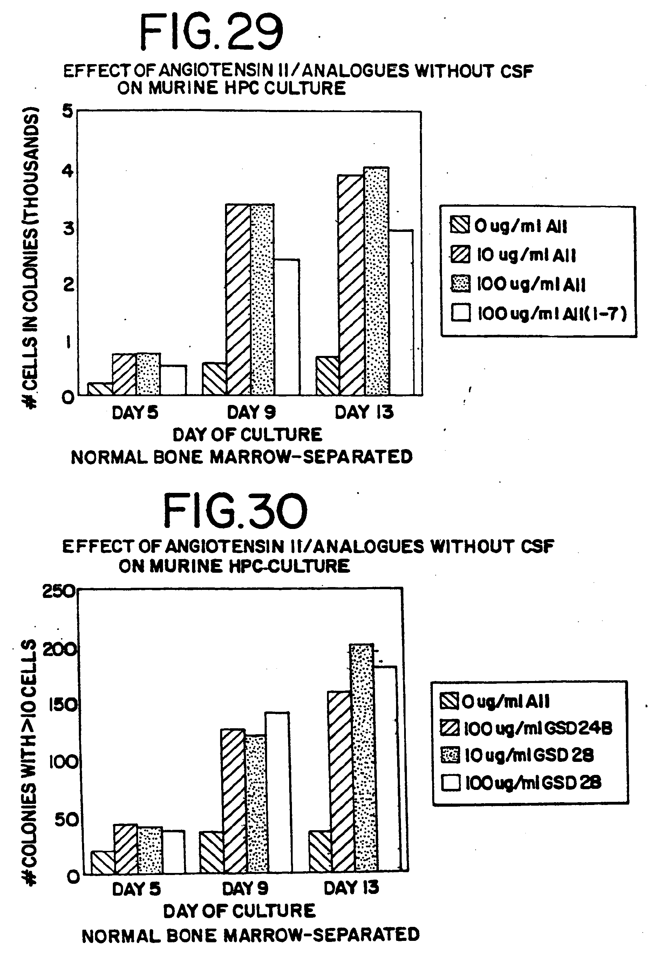

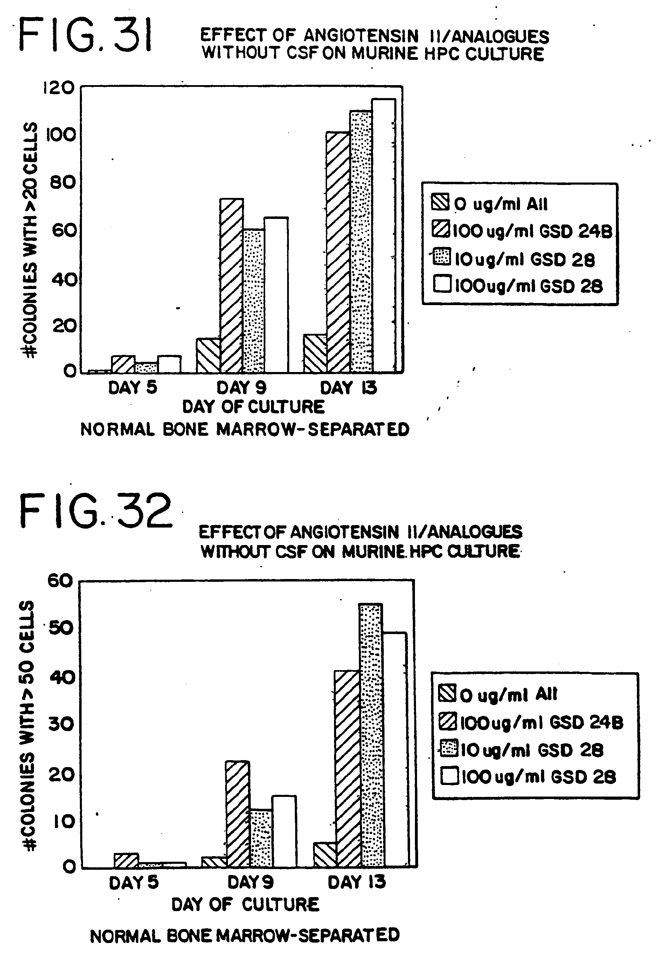

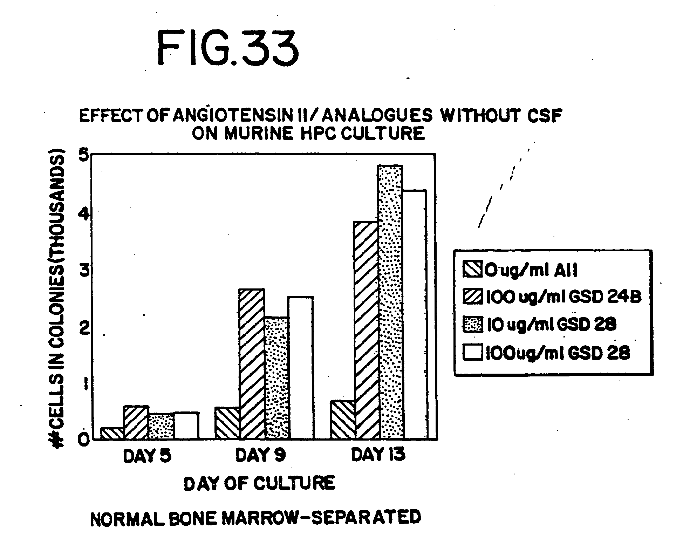

The enriched HPC cell fractions were diluted into a semi-solid medium containing 0.9% methylcellulose in alpha minimal essential medium (alpha MEM), 30% fetal calf serum, 1% bovine serum albumin, 10⁻⁴ M 2-mercaptoethanol, 2 mM L-glutamine, and 2% conditioned medium. The conditioned medium was supernatant from splenocyte cultures (1 x 10⁶ cells/ml) incubated for 48 hours in RPMI 1640 containing 10 µg/ml pokeweed mitogen (PWM), 10% FCS, and antibiotics. Various concentrations of AII, between 0 and 100 µg/ml, or AII analogues or AII fragments were added in a small volume to the wells of microtiter dishes, which contained approximately either 3,000 cells per well or 30,000 cells per well. The cells were incubated at 37°C and 5% CO₂ for 14 days. Microscopic evaluation of the cells was performed at various days after initiation of incubation, and the results are summarized in Figures 23-33. The results clearly demonstrate that HPC colony size increases proportionately with exposure to increased concentrations of AII, AII analogues and AII fragments, and thus that AII increases HPC proliferation in the absence of colony stimulating factors.

## Claims

1. An in vitro method of accelerating the proliferation of hematopoietic pluripotent progenitor cells or hematopoietic lineage-specific cells, comprising contacting said cells with an amount effective to accelerate proliferation of said cells of at least one active agent comprising a peptide whose sequence is selected from the group consisting of Val-Tyr-Ile-His-Pro-Phe (SEQ ID NO:3), Asp-Arg-Val-Tyr-Ile-His-Pro (SEQ ID NO:4), Arg-Val-Tyr-Ile-His-Pro (SEQ ID NO:5), Val-Tyr-Ile-His-Pro (SEQ ID NO:6), Ile-His-Pro-Phe (SEQ ID NO:7), Asp-Arg-Val-Tyr-Ile-His (SEQ ID NO:8), Asp-Arg-Val-Tyr-Ile (SEQ ID NO:9), Asp-Arg-Val-Tyr (SEQ ID NO:10), Asp-Arg-Val (SEQ ID NO:11), Asp-Arg-Pro-Tyr-Ile-His-Pro-Phe (SEQ ID NO:31) and Asp-Arg-Val-Tyr-Ile-His-Pro-Ile (SEQ ID NO:38).

2. An in vitro method of accelerating the differentiation of hematopoietic pluripotent progenitor cells or hematopoietic lineage-specific cells, comprising contacting said cells with an amount effective to accelerate proliferation of said cells of at least one active agent comprising a peptide whose sequence is selected from the group consisting of Val-Tyr-Ile-His-Pro-Phe (SEQ ID NO:3), Asp-Arg-Val-Tyr-Ile-His-Pro (SEQ ID NO:4), Arg-Val-Tyr-Ile-His-Pro (SEQ ID NO:5), Val-Tyr-Ile-His-Pro (SEQ ID NO:6), Ile-His-Pro-Phe (SEQ ID NO:7), Asp-Arg-Val-Tyr-Ile-His (SEQ ID NO:8), Asp-Arg-Val-Tyr-Ile (SEQ ID NO:9), Asp-Arg-Val-Tyr (SEQ ID NO: 10), Asp-Arg-Val (SEQ ID NO:11), Asp-Arg-Pro-Tyr-Ile-His-Pro-Phe (SEQ ID NO:31) and Asp-Arg-Val-Tyr-Ile-His-Pro-Ile (SEQ ID NO:38).

3. The method of claim 2, wherein the hematopoietic pluripotent progenitor cells or lineage-specific cells are selected from the group consisting of macrophages and monocytes.

4. The method of any one of claims 1-3 wherein the concentration of active agent is between about 0.1 ng/ml and about 1.0 mg/ml.

5. The use of an active agent comprising a peptide whose sequence is selected from the group consisting of Val-Tyr-Ile-His-Pro-Phe (SEQ ID NO:3), Asp-Arg-Val-Tyr-Ile-His-Pro (SEQ ID NO:4), Arg-Val-Tyr-Ile-His-Pro (SEQ ID NO:5), Val-Tyr-Ile-His-Pro (SEQ ID NO:6), Ile-His-Pro-Phe (SEQ ID NO:7), Asp-Arg-Val-Tyr-Ile-His (SEQ ID NO:8), Asp-Arg-Val-Tyr-Ile (SEQ ID NO:9), Asp-Arg-Val-Tyr (SEQ ID NO:10), Asp-Arg-Val (SEQ ID NO:11), Asp-Arg-Pro-Tyr-Ile-His-Pro-Phe (SEQ ID NO:31) and Asp-Arg-Val-Tyr-Ile-His-Pro-Ile (SEQ ID NO:38) for the preparation of a medicament for the treatment of an oncology patient, wherein the proliferation or differentiation of hematopoietic pluripotent progenitor cells or lineage-specific cells is accelerated.

6. The use of claim 5, wherein the treatment involves hematopoietic pluripotent progenitor cell transplantation therapy.

7. The use of claim 5 wherein the active agent is used for augmenting anti-tumor immunity.

8. The use of an active agent comprising a peptide whose sequence is selected from the group consisting of Val-Tyr-Ile-His-Pro-Phe (SEQ ID NO:3), Asp-Arg-Val-Tyr-Ile-His-Pro (SEQ ID NO:4), Arg-Val-Tyr-Ile-His-Pro (SEQ ID NO:5), Val-Tyr-Ile-His-Pro (SEQ ID NO:6), Ile-His-Pro-Phe (SEQ ID NO:7), Asp-Arg-Val-Tyr-Ile-His (SEQ ID NO:8), Asp-Arg-Val-Tyr-Ile (SEQ ID NO:9), Asp-Arg-Val-Tyr (SEQ ID NO:10), Asp-Arg-Val (SEQ ID NO:11), Asp-Arg-Pro-Tyr-Ile-His-Pro-Phe (SEQ ID NO:31) and Asp-Arg-Val-Tyr-Ile-His-Pro-Ile (SEQ ID NO:38) for the preparation of a medicament for the treatment of one or more of viral infections and microbial infections wherein the proliferation or differentiation of hematopoietic pluripotent progenitor cells or lineage-specific cells is accelerated.

9. The use of any one of claims 5-8 wherein the dosage of active agent is between about 0.1 ng/kg and about 1.0 mg/kg.

10. The use of hematopoietic pluripotent progenitor cells cultured according to the method of any one of claims 1-4 for the preparation of a medicament for autologous transplantation to reconstitute a depleted hematopoietic system.

## Patentansprüche

1. In-vitro-Verfahren zur Beschleunigung der Proliferation von hämatopoetischen, pluripotenten Vorläuferzellen oder von für die hämatopoetische Linie spezifischen Zellen, umfassend:
In-Kontakt-Bringen besagter Zellen mit einer für das Beschleunigen der Proliferation der Zellen wirksamen Menge mindestens eines Wirkstoffs, welcher ein Peptid umfasst, dessen Sequenz aus der Gruppe ausgewählt ist, die aus Val-Tyr-Ile-His-Pro-Phe (SEQ ID NO: 3), Asp-Arg-Val-Tyr-Ile-His-Pro (SEQ ID NO: 4), Arg-Val-Tyr-Ile-His-Pro (SEQ ID NO: 5), Val-Tyr-Ile-His-Pro (SEQ ID NO: 6), Ile-His-Pro-Phe (SEQ ID NO: 7), Asp-Arg-Val-Tyr-Ile-His (SEQ ID NO: 8), Asp-Arg-Val-Tyr-Ile (SEQ ID NO: 9), Asp-Arg-Val-Tyr (SEQ ID NO: 10), Asp-Arg-Val (SEQ ID NO: 11), Asp-Arg-Pro-Tyr-Ile-His-Pro-Phe (SEQ ID NO: 31) und Asp-Arg-Val-Tyr-Ile-His-Pro-Ile (SEQ ID NO: 38) besteht.

2. In-vitro-Verfahren zur Beschleunigung der Differenzierung von hämatopoetischen, pluripotenten Vorläuferzellen oder von für die hämatopoetische Linie spezifischen Zellen, umfassend:
In-Kontakt-Bringen besagter Zellen mit einer für das Beschleunigen der Proliferation der Zellen wirksamen Menge mindestens eines Wirkstoffs, welcher ein Peptid umfasst, dessen Sequenz aus der Gruppe ausgewählt ist, die aus Val-Tyr-Ile-His-Pro-Phe (SEQ ID NO: 3), Asp-Arg-Val-Tyr-Ile-His-Pro (SEQ ID NO: 4), Arg-Val-Tyr-Ile-His-Pro (SEQ ID NO: 5), Val-Tyr-Ile-His-Pro (SEQ ID NO: 6), Ile-His-Pro-Phe (SEQ ID NO: 7), Asp-Arg-Val-Tyr-Ile-His (SEQ ID NO: 8), Asp-Arg-Val-Tyr-Ile (SEQ ID NO: 9), Asp-Arg-Val-Tyr (SEQ ID NO: 10), Asp-Arg-Val (SEQ ID NO: 11), Asp-Arg-Pro-Tyr-Ile-His-Pro-Phe (SEQ ID NO: 31) und Asp-Arg-Val-Tyr-Ile-His-Pro-Ile (SEQ ID NO: 38) besteht.

3. Verfahren nach Anspruch 2, wobei die hämatopoetischen, pluripotenten Vorläuferzellen oder die für die Linie spezifischen Zellen aus der Gruppe ausgewählt sind, die aus Makrophagen und Monozyten besteht.

4. Verfahren nach einem der Ansprüche 1-3, wobei die Konzentration des Wirkstoffs zwischen ungefähr 0,1 ng/ml und ungefähr 1,0 mg/ml liegt.

5. Verwendung eines Wirkstoffs, welcher ein Peptid umfasst, dessen Sequenz aus der Gruppe ausgewählt ist, die aus Val-Tyr-Ile-His-Pro-Phe (SEQ ID NO: 3), Asp-Arg-Val-Tyr-Ile-His-Pro (SEQ ID NO: 4), Arg-Val-Tyr-Ile-His-Pro (SEQ ID NO: 5), Val-Tyr-Ile-His-Pro (SEQ ID NO: 6), Ile-His-Pro-Phe (SEQ ID NO: 7), Asp-Arg-Val-Tyr-Ile-His (SEQ ID NO: 8), Asp-Arg-Val-Tyr-Ile (SEQ ID NO: 9), Asp-Arg-Val-Tyr (SEQ ID NO: 10), Asp-Arg-Val (SEQ ID NO: 11), Asp-Arg-Pro-Tyr-Ile-His-Pro-Phe (SEQ ID NO: 31) und Asp-Arg-Val-Tyr-Ile-His-Pro-Ile (SEQ ID NO: 38) besteht, zur Herstellung eines Medikaments für die Behandlung eines Onkologiepatienten, wobei die Proliferation oder Differenzierung von hämatopoetischen, pluripotenten Vorläuferzellen oder von für die Linie spezifischen Zellen beschleunigt wird.

6. Verwendung nach Anspruch 5, wobei die Behandlung eine Transplantationstherapie mit hämatopoetischen, pluripotenten Vorläuferzellen umfasst.

7. Verwendung nach Anspruch 5, wobei der Wirkstoff zur Steigerung der Anti-Tumor-Immunität verwendet wird.

8. Verwendung eines Wirkstoffs, welcher ein Peptid umfasst, dessen Sequenz aus der Gruppe ausgewählt ist, die aus Val-Tyr-Ile-His-Pro-Phe (SEQ ID NO: 3), Asp-Arg-Val-Tyr-Ile-His-Pro (SEQ ID NO: 4), Arg-Val-Tyr-Ile-His-Pro (SEQ ID NO: 5), Val-Tyr-Ile-His-Pro (SEQ ID NO: 6), Ile-His-Pro-Phe (SEQ ID NO: 7), Asp-Arg-Val-Tyr-Ile-His (SEQ ID NO: 8), Asp-Arg-Val-Tyr-Ile (SEQ ID NO: 9), Asp-Arg-Val-Tyr (SEQ ID NO: 10), Asp-Arg-Val (SEQ ID NO: 11), Asp-Arg-Pro-Tyr-Ile-His-Pro-Phe (SEQ ID NO: 31) und Asp-Arg-Val-Tyr-Ile-His-Pro-Ile (SEQ ID NO: 38) besteht, zur Herstellung eines Medikaments für die Behandlung eines oder mehreren von viralen Infektionen und mikrobiellen Infektionen, wobei die Proliferation oder Differenzierung von hämatopoetischen, pluripotenten Vorläuferzellen oder von für die Linie spezifischen Zellen beschleunigt wird.

9. Verwendung nach einem der Ansprüche 5-8, wobei die Dosierung des Wirkstoffs zwischen ungefähr 0,1 ng/kg und ungefähr 1,0 mg/kg liegt.

10. Verwendung hämatopoetischer, pluripotenter Vorläuferzellen, die gemäß dem Verfahren nach einem der Ansprüche 1-4 kultiviert wurden, zur Herstellung eines Medikaments für die autologe Transplantation, um ein verarmtes hämatopoetisches System wiederherzustellen.

## Revendications

1. Procédé in vitro pour accélérer la prolifération des cellules hématopoïétiques progénitrices pluripotentes ou des cellules spécifiques d'une lignée hématopoïétique, comprenant la mise en contact desdites cellules avec une quantité efficace pour accélérer la prolifération desdites cellules d'au moins un agent actif comprenant un peptide dont la séquence est choisie dans le groupe consistant en Val-Tyr-Ile-His-Pro-Phe (SEQ ID NO: 3), Asp-Arg-Val-Tyr-Ile-His-Pro (SEQ ID NO: 4), Arg-Val-Tyr-Ile-His-Pro (SEQ ID NO: 5), Val-Tyr-Ile-His-Pro (SEQ ID NO: 6), Ile-His-Pro-Phe (SEQ ID NO: 7), Asp-Arg-Val-Tyr-Ile-His (SEQ ID NO: 8), Asp-Arg-Val-Tyr-Ile (SEQ ID NO: 9), Asp-Arg-Val-Tyr (SEQ ID NO: 10), Asp-Arg-Val (SEQ ID NO: 11), Asp-Arg-Pro-Tyr-Ile-His-Pro-Phe (SEQ ID NO: 31) et Asp-Arg-Val-Tyr-Ile-His-Pro-Ile (SEQ ID NO: 38).

2. Procédé in vitro pour accélérer la différenciation des cellules hématopoïétiques progénitrices pluripotentes ou des cellules spécifiques d'une lignée hématopoïétique, comprenant la mise en contact desdites cellules avec une quantité efficace pour accélérer la prolifération desdites cellules d'au moins un agent actif comprenant un peptide dont la séquence est choisie dans le groupe consistant en Val-Tyr-Ile-His-Pro-Phe (SEQ ID NO: 3), Asp-Arg-Val-Tyr-Ile-His-Pro (SEQ ID NO: 4), Arg-Val-Tyr-Ile-His-Pro (SEQ ID NO: 5), Val-Tyr-Ile-His-Pro (SEQ ID NO: 6), Ile-His-Pro-Phe (SEQ ID NO: 7), Asp-Arg-Val-Tyr-Ile-His (SEQ ID NO: 8), Asp-Arg-Val-Tyr-Ile (SEQ ID NO: 9), Asp-Arg-Val-Tyr (SEQ ID NO: 10), Asp-Arg-Val (SEQ ID NO: 11), Asp-Arg-Pro-Tyr-Ile-His-Pro-Phe (SEQ ID NO: 31) et Asp-Arg-Val-Tyr-Ile-His-Pro-Ile (SEQ ID NO: 38).

3. Procédé selon la revendication 2, dans lequel les cellules hématopoïétiques progénitrices pluripotentes ou spécifiques d'une lignée sont choisies dans le groupe consistant en les macrophages et les monocytes.

4. Procédé selon l'une quelconque des revendications 1-3 dans lequel la concentration d'agents actifs se situe entre environ 0,1 ng/ml et environ 1,0 mg/ml.

5. Utilisation d'un agent actif comprenant un peptide dont la séquence est choisie dans le groupe consistant en Val-Tyr-Ile-His-Pro-Phe (SEQ ID NO: 3), Asp-Arg-Val-Tyr-Ile-His-Pro (SEQ ID NO: 4), Arg-Val-Tyr-Ile-His-Pro (SEQ ID NO: 5), Val-Tyr-Ile-His-Pro (SEQ ID NO: 6), Ile-His-Pro-Phe (SEQ ID NO: 7), Asp-Arg-Val-Tyr-Ile-His (SEQ ID NO: 8), Asp-Arg-Val-Tyr-Ile (SEQ ID NO: 9), Asp-Arg-Val-Tyr (SEQ ID NO: 10), Asp-Arg-Val (SEQ ID NO: 11), Asp-Arg-Pro-Tyr-Ile-His-Pro-Phe (SEQ ID NO: 31) et Asp-Arg-Val-Tyr-Ile-His-Pro-Ile (SEQ ID NO: 38) pour la préparation d'un médicament pour le traitement d'un patient en oncologie, tandis que la prolifération ou la différenciation des cellules hématopoïétiques progénitrices pluripotentes ou spécifiques d'une lignée est accélérée.

6. Utilisation selon la revendication 5, dans laquelle le traitement fait intervenir une thérapie par transplantation de cellules hématopoïétiques progénitrices pluripotentes.

7. Utilisation selon la revendication 5, dans laquelle l'agent actif est utilisé pour augmenter l'immunité anti-tumorale.

8. Utilisation d'un agent actif comprenant un peptide dont la séquence est choisie dans le groupe consistant en Val-Tyr-Ile-His-Pro-Phe (SEQ ID NO: 3), Asp-Arg-Val-Tyr-Ile-His-Pro (SEQ ID NO: 4), Arg-Val-Tyr-Ile-His-Pro (SEQ ID NO: 5), Val-Tyr-Ile-His-Pro (SEQ ID NO: 6), Ile-His-Pro-Phe (SEQ ID NO: 7), Asp-Arg-Val-Tyr-Ile-His (SEQ ID NO: 8), Asp-Arg-Val-Tyr-Ile (SEQ ID NO: 9), Asp-Arg-Val-Tyr (SEQ ID NO: 10), Asp-Arg-Val (SEQ ID NO: 11), Asp-Arg-Pro-Tyr-Ile-His-Pro-Phe (SEQ ID NO: 31) et Asp-Arg-Val-Tyr-Ile-His-Pro-Ile (SEQ ID NO: 38) pour la préparation d'un médicament pour le traitement d'une ou plusieurs infections virales et infections microbiennes, tandis que la prolifération ou la différenciation des cellules hématopoïétiques progénitrices pluripotentes ou spécifiques d'une lignée est accélérée.

9. Utilisation selon l'une quelconque des revendications 5-8, dans laquelle le dosage d'agent actif est entre environ 0,1 ng/kg et environ 1,0 mg/kg.

10. Utilisation de cellules progénitrices hématopoïétiques pluripotentes cultivées conformément au procédé selon l'une quelconque des revendications 1-4 pour la préparation d'un médicament pour transplantation autologue pour la reconstitution d'un système hématopoïétique épuisé.
